# EUROPEAN PATENT SPECIFICATION

(11) **EP 2 419 030 B1**
(45) Date of publication and mention of the grant of the patent: **11.01.2017**
(21) Application number: 10715054.2
(22) Date of filing: 15.04.2010
(51) Int. Cl.: A61B 17/70, A61F 2/44

(54) **Intervertebral implant**
Zwischenwirbelimplantat
Implant intervertébral

(30) Priority: 15.04.2009 US 169461 P
(43) Date of publication of application: 22.02.2012
(73) Proprietor: Synthes GmbH, 4436 Oberdorf (CH)
(72) Inventor: DONNER, Thomas, Thibodaux LA 70301 (US); LAURENCE, Lawton, West Chester PA 19382 (US); SINGHATAT, Wamis, West Chester, PA 19380 (US); SCHOENLY, Jared, West Chester, PA 19380 (US); EVANS, David, West Chester, PA 19380 (US); MILLER, William, West Chester, PA 19380 (US); SCHMURA, Kurt, West Chester, PA 19380 (US)
(74) Representative: Klunker . Schmitt-Nilson . Hirsch
(86) International application number: PCT/US2010/031244
(87) International publication number: WO 2010/121028

(56) References cited:
- WO-A1-2009/131955
- WO-A2-02/13732
- WO-A2-2007/098288
- WO-A2-2009/004625
- FR-A1- 2 848 408
- FR-A1- 2 916 956
- US-A- 4 790 303
- US-A- 5 899 905
- US-A1- 2004 210 219

## Description

The present disclosure relates generally to orthopedics, and in particular relates to fixation systems, intervertebral implants, and associated surgical methods and procedures for using same.

### BACKGROUND

Spinal fixation systems such as pedicle screw and rod constructs are commonly used to promote fusion between interverterbral bodies. The insertion of pedicle screws typically requires a linear "line-of-approach" trajectory that is aligned with the longitudinal axis of the screw, in order to accommodate the access and delivery instruments. Similarly, anchors such as bone screws may be used to directly fix intervertebral implants to vertebral bodies, typically requiring the insertion of several screws at unique angles oblique to the sagittal and/or transverse plane, and thus mulitple lines-of-approach. However, in a variety of surgical situations, achieving a desired trajectory for screw insertion can be difficult due to the patient's anatomy obstructing a linear line-of-approach. For example, medially-directed placement of pedicle screws into the sacrum is desirable to prevent screw loosening and/or pullout, but can be prohibited due to the iliac crest obstructing the linear line-of-approach.

In addition to the above-discussed linear line-of-approach problems, limitations of the fixation anchors themselves can further limit spinal fixation treatment approaches. For example, unilateral spinal fixation procedures, wherein a pedicle screw and rod construct is placed on a single side of the spine, provide advantages such as limiting surgical site morbidity and shortening surgical time when compared with standard bilateral fixation procedures wherein the construct is placed on both sides of the spine and interconnected. However, unilateral fusion constructs typically exhibit decreased mechanical rigidity in comparison to bilateral constructs, for example due to lower torsional and/or rotational rigidty and weaker resistance to screw pullout forces under physiologic loading when compared to typical bilateral constructs. As a result, unilateral fixation procedures are rarely performed in lieu of bilateral fixation procedures.

What is therefore desirable are spinal fixation systems that allow for the creation of rigid constructs when the linear line-of-approach for insertion of fixation anchors is unavailable and/or undesirable (e.g., when multiple anchors are required), while at the same time providing increased rigidity and robustness to spinal constructs such as those used in unilateral fusion procedures.

FR 2 916-956 A1 and WO 02/13732 A2 disclose intervertebral implants having an implant body and curved fixation members to fix the intervertebral implant to adjacent vertebrae. The implant body has apertures to receive the curved apertures, the apertures extending in an angled but linear manner through the posterior portion of the implant body.

### SUMMARY

Arcuate fixation members, hereinafter also referred to as curved fixation members, with varying configurations and/or features are disclosed, along with additional components for use therewith in disclosed fixation systems and intervertebral implant systems. The arcuate fixation members may be of varying lengths, cross sectional geometries, and/or cross sectoinal areas, and may be configured with various features such as heads configured to accept other fixation system components, tabs to allow arcuate fixation member-in-arcuate fixation member or fixation anchor-in-arcuate fixation member configurations. Fixation systems or intervertebral implant systems utilizing arcuate fixation members are particularly suitable when a linear line-of-approach for delivering fixation members is undesirable.

### BRIEF DESCRIPTION OF THE DRAWINGS

The foregoing summary, as well as the following detailed description of the preferred embodiments of the application, will be better understood when read in conjunction with the appended drawings. For the purposes of illustrating the arcuate fixation member systems and methods, there are shown in the drawings preferred embodiments. It should be understood, however, that the instant application is not limited to the precise arrangements and/or instrumentalities illustrated in the drawings, in which:

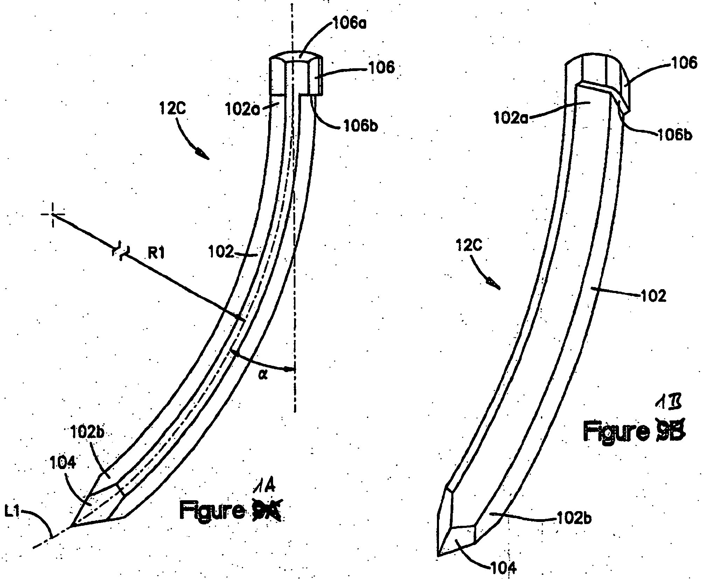

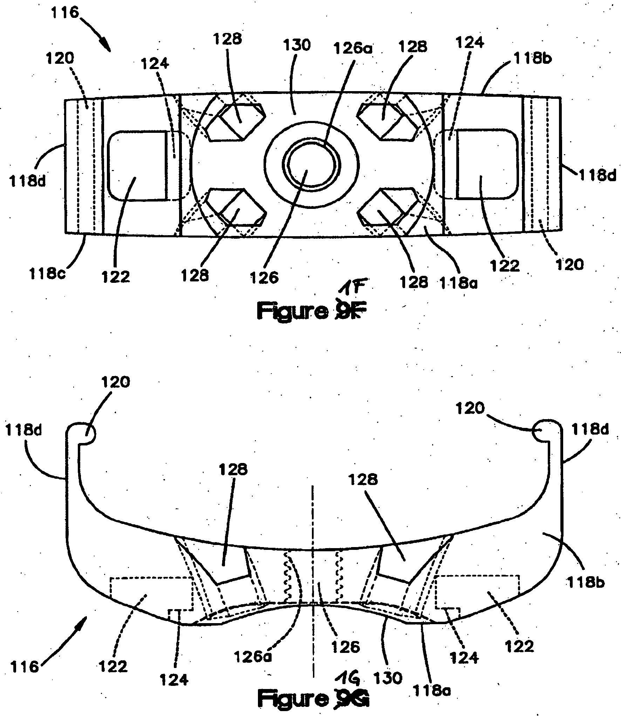

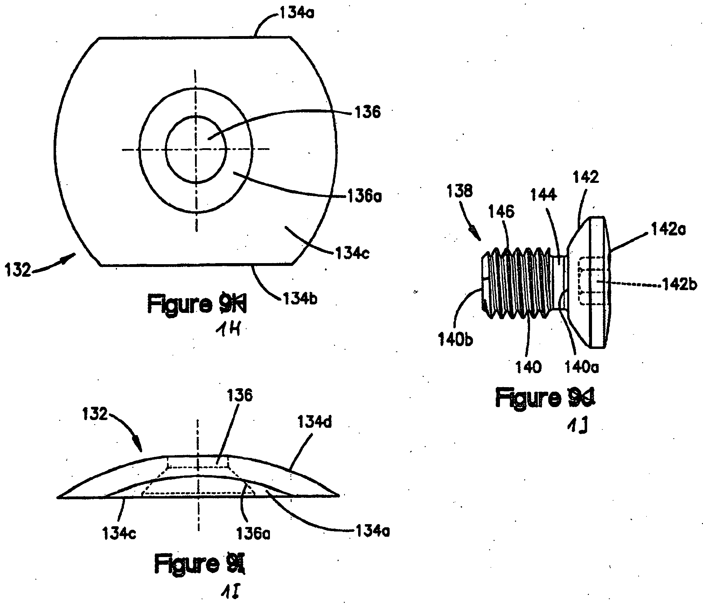

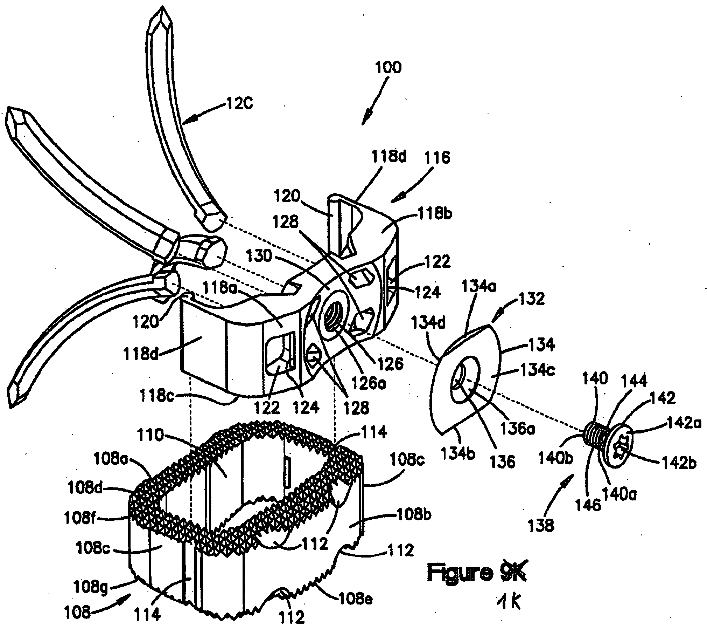

Figures 1A is a side elevation view of an arcuate fixation member constructed in accordance with an embodiment;
Figure 1B is a perspective view of the arcuate fixation member illustrated in Figure 1A;

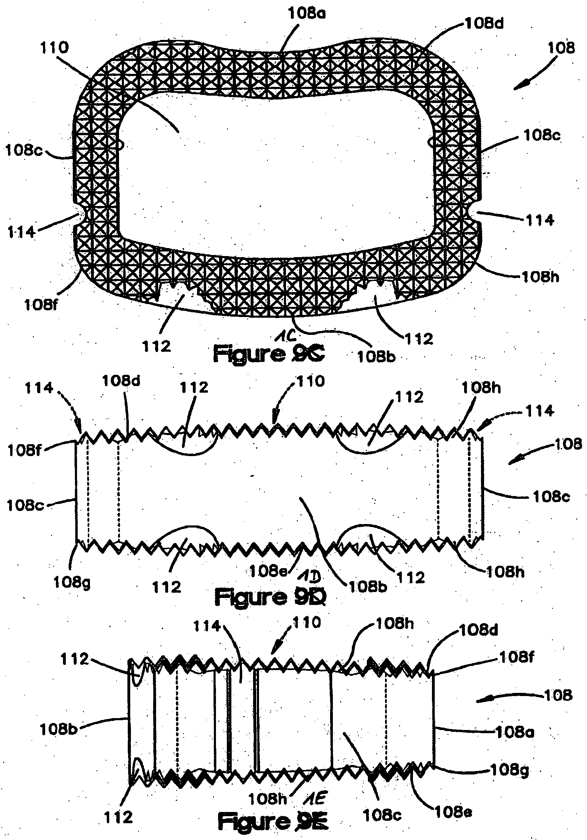

Figure 1C is a top elevation view of an embodiment of an intervertebral implant body for use with arcuate fixation members;
Figure 1D is a front elevation view of the intervertebral implant body illustrated in Figure 1C;
Figure 1E is a side elevation view of the intervertebral implant body illustrated in Figure 1C;
Figure 1E is a side elevation view of the intervertebral implant body illustrated in Figure 1C;
Figure 1F is a front elevation view of an embodiment of a fixation plate for use with the intervertebral implant body illustrated in Figures 1C to 1E;
Figure 1G is a top elevation view of the fixation plate illustrated in Figure 1F;
Figure 1H is a front elevation view of a blocking plate for use with the fixation plate illustrated in Figures 1F and 1G;
Figure 1I is a top elevation view of the blocking plate illustrated in Figure 1H;
Figure 1J is a side elevation view of a locking screw for use with the fixation plate illustrated in Figures 1F and 1G;
Figure 1K is an exploded view of an assembly utilizing the components illustrated in Figures 1A to 1J;
Figure 1L is a perspective view of an assembly utilizing the components illustrated in Figures 1A to 1J;
Figure 1M is a side elevation view of the assembly illustrated in Figure 1L inserted into an intervertebral space;
Figure 2A is a top elevation view of another embodiment of an intervertebral implant body for use with arcuate fixation members;
Figure 2B is a front elevation view of the intervertebral implant body illustrated in Figure 2A;
Figure 2C is a top elevation view of another embodiment of an intervertebral implant body for use with arcuate fixation members;
Figure 2D is a front elevation view of the intervertebral implant body illustrated in Figure 2C;
Figure 2E is a front elevation view of an embodiment of a fixation plate for use with the intervertebral implant bodies illustrated in Figures 2A to 2D;
Figure 2F is a top elevation view of the fixation plate illustrated in Figure 2E;
Figure 2G is a front elevation view of a blocking plate for use with the fixation plate illustrated in Figures 2E and 2F;
Figure 2H is a top elevation view of the blocking plate illustrated in Figure 10G;
Figure 2I is an exploded view of an assembly utilizing the components illustrated in Figures 2A to 2H;
Figure 2J is a perspective view of an assembly utilizing the components illustrated in Figures 2A to 2H;
Figure 3A is a front elevation view of a ratchet plate for use with the fixation plate illustrated in Figures 2E and 2F;
Figure 3B is a top elevation view of the ratchet plate illustrated in Figure 3A;
Figure 3C is a side elevation view of the ratchet plate illustrated if Figure 3A;
Figures 3D is a perspective view of an arcuate fixation member constructed in accordance with another embodiment;
[0052] Figure 3E is an exploded view of an assembly utilizing the components illustrated in Figures 1J, 2E, 2F, and 3A to 3D;
Figure 3F is a perspective view of the assembly illustrated in Figure 3E;
Figures 3G and 3H illustrate a side elevation cross sectional view of the assembly illustrated in Figure 3E;
Figure 4A is a perspective view of an arcuate fixation member and a complimentary screw constructed in accordance with another embodiment;
Figure 4B is a perspective view of the arcuate fixation member and the complimentary screw in a mated configuration;
Figure 4C is a perspective view of an arcuate groove configured to receive the components illustrated in Figures 4A and 4B;
Figure 4D is a perspective view of an assembly utilizing the components illustrated in Figures 4A to 4C;
Figure 5A is an exploded view of an assembled components of an embodiment of a delivery instrument;
Figure 5B is a perspective view of an embodiment of a guide ring for use with the components illustrated in Figure 5A;
Figure 5C is a perspective view of an embodiment of a drive head for use with the components illustrated in Figure 5A
Figure 5D is a side elevation view of an assembly utilizing components illustrated in Figures 5A to 5C in combination with the assembly illustrated in Figure 1L;
Figure 5E is a perspective view of the assembly illustrated in Figure 5D;
Figure 5F is a perspective view of another embodiment of the delivery instrument illustrated in Figures 5A to 5E;
Figure 6A is a top elevation view of assembled components of another embodiment of a delivery instrument;
Figure 6B is a perspective view of a component illustrated in Figure 6A;
Figure 6C is a perspective view of another of the components illustrated in Figure 6A;
Figure 6D is a perspective view of components illustrated in Figure 6A; and
Figure 6E is a perspective view of another embodiment of the delivery instrument illustrated in Figures 6A to 6D.

### DETAILED DESCRIPTION OF ILLUSTRATIVE EMBODIMENTS

Certain terminology is used in the following description for convenience only and is not limiting. The words "right", "left", "top" and "bottom" designate directions in the drawings to which reference is made. The words "inwardly" and "outwardly" refer to directions toward and away from, respectively, the geometric center of the device and designated parts thereof. The words, "anterior", "posterior", "superior", "inferior", "lateral", "medial", "sagittal", "axial", "coronal," "cranial," "caudal" and related words and/or phrases designate preferred positions and orientations in the human body to which reference is made and are not meant to be limiting.

The words "arcuate" and "curved" as used herein refer generally to the varying physical geometry of an object along an axis coincident to the object, for example the deviation from straightness of the body of an arcuate fixation member along a central longitudinal axis defined with the body of the object between its proximal and distal ends. Generally, with reference to a straight axis projected from a first end of such an object, as distance from the first end of the object increases along the central longitudinal axis of the object, distance between the central longitudinal axis of the object and the straight axis increases more or less continuously, so that the body of the object defined along its central longitudinal axis takes on a curved or arcuate shape. The resulting curvature of the central longitudinal axis may exhibit a constant or uniform radius with respect to a point in space defined remotely from the body of the object. Alternatively, a non-uniform or varying radius of curvature may be defined. The curvature of the body of the object defined by the longitudinal axis may also vary in direction with respect to a Cartesian coordinate system. The curvature may be uniformly distributed along the body of the object, for example between the proximal and distal ends of the object, or may be localized within one or more distinct segments of the body of the object. The curvature of the object may be significantly smooth and continuous along its central longitudinal axis, may be defined by a series of straight interconnected segments where each successive segment defines an increasing angle between the central longitudinal axis of the body of the object and the straight axis, or any combination thereof.

The terminology intended to be non-limiting includes the above-listed words, derivatives thereof and words of similar import.

Described herein are arcuate fixation members with varying configurations and/or features, along with additional components for use therewith in intervertebral implant systems. Applications of intervertebral implants systems utilizing arcuate fixation members could include, but are not limited to, fixation of the endplate components of a total disc replacement to vertebral bodies, lateral mass fixation in the cervical spine, direct fixation of an intervertebral implant to vertebral bodies, fixation into osteoporotic bone, anchor-in-anchor fixation into underlying bone, securing auxilary fixation devices to underlying bone, and the like. The use of systems and/or methods utilizing arcuate fixation members disclosed herein are particularly suitable when a linear line-of approach for delivering fixation member is undesirable. It should be noted that the physical characteristics of the arcuate fixation members disclosed herein may cause them to be alternately described as arcuate blades, arcuate pins, arcuate nails, or other terms of similar descriptive import.

Referring now to Figures 1A to 1M, an example embodiment of an intervertebral implant system 100 comprising an arcuate fixation member 12C, an intervertebral implant body 108, a fixation plate 116, a blocking plate 132, and a locking screw 138 is illustrated. As will become appreciated from the description below, one or more fixation members 12C may be utilized to securely anchor an assembled configuration of intervertebral implant system 100 within an intervertebral space between adjacent vertebral bodies. Unless otherwise indicated, the intervertebral implant system 100 and its components can be manufactured from any suitable biocompatible material known in the art including but not limited to titanium, titanium alloy such as TAN, stainless steel, reinforced plastics, allograft bone, and the like.

The arcuate fixation member 12C includes a body 102 defining a proximal end 102a and a distal end 102b opposite the proximal end. The distal end 102b may comprise a tip 104 configured to cut into underlying structure or bore. The body 102 may further define an intermediate portion between the proximal end 102a and the distal end 102b that is curved along a central curved axis L1. In an embodiment, the intermediate portion is curved along substantially the entire length of the body 102 between the proximal end 102a and the distal end 102b. Alternatively, one or more distinct portions of the intermediate portion between the proximal end 102a and the distal end 102b may be curved (not shown).

In an embodiment, the intermediate portion is curved along the central curved axis L1 in accordance with a uniform radius of curvature R1. Alternatively, the intermediate portion may define a non-uniform radius of curvature along the central curved axis L1. In a preferred embodiment, the curvature of the intermediate portion may be smooth and continuous. Alternatively, the curvature of the intermediate portion may be defined by a series of substantially straight sections (not shown), with each substantially straight section aligned along an individual longitudinal axis corresponding to the individual section, where the magnitude of an angle α with respect to a perpendicular reference axis extended from the proximal end 102a increases in magnitude with the distance of each subsequent straight section from the proximal end 102a.

The arcuate fixation member 12C may have a head 106 defined at the proximal end 102a of the body 102. The head 106 may extend radially outward from the proximal end 102a of the body 102 in a direction perpendicular to the longitudinal axis L1. In an example embodiment, the head 106 may extend from the body 102 in a direction generally opposite from the direction of curvature of the body 102, as depicted in Figures 1A and 1B. alternative embodiments, the head 106 may extend from the body 102 in a direction generally towards the direction of curvature of the body 102. The head may define an upper surface 106a configured for multi-angular engagement with a complementary surface of a delivery instrument, and a lower surface 106b opposite the upper surface and configured to engage another component of the intervertebral implant system 100, for example the fixation plate 116, when the arcuate fixation member 12C is in a fully inserted position.

The intervertebral implant body 108 is defined by a posterior side 108a, an anterior side 108b opposite the posterior side, lateral sides 108c, an upper surface 108d, and a lower surface 108e opposite the upper surface. In an example embodiment, a portion of the posterior side 108a between the lateral sides 108c may be curved inwardly in the direction of the anterior side 108b, defining a rounded, generally rectangular kidney-like footprint, as depicted in Figure 1C. In an alternative embodiment, a portion of the posterior side 108a between the lateral sides 108c may be curved outwardly in a direction away from the anterior side 108b (not shown). In another alternative embodiment, the posterior side 108a may be substantially straight between the lateral sides 108c defining a rounded, generally rectangular footprint (not shown). The intervertebral implant body may have a central bore 110 formed therethrough, the shape of which substantially conforms to the footprint of the intervertebral implant body 108 (*e.g*., a rounded, generally rectangular kidney-like footprint, or arounded, generally rectangular footprint, depending upon the geometry of the posterior side 108a). The central bore 110 can be filled with bone growth inducing substances to allow bony ingrowth and to assist in fusion between the intervertebral implant body 108 and adjacent vertebral bodies.

In an example embodiment of the intervertebral implant body 108, the upper and lower surfaces 108d and 108e may have gripping features 108h such as teeth, spikes, or similar structures, formed thereon and configured to facilitate gripping engagement between the upper and lower surfaces 108d and 108e and the end plates of adjacent vertebral bodies. The teeth 112 may be pyramidal, saw toothed or other similar shapes. In alternative embodiments of the intervertebral implant body 108, portions of and/or the entirety of the upper and lower surfaces 108d and 108e may be substantially smooth and devoid of any protrusions. Upper and lower edges 108f and 108g, defined where the upper and lower surfaces 108d and 108e intersect with the posterior, anterior, and lateral sides 108a, 108b, and 108c respectively around the outer perimeter of the intervertebral implant body 108, may be rounded (not shown). In an example embodiment, the upper and lower edges 108f and 108b may be rounded using a uniform radius of curvature around the perimeter of the implant. In an alternative embodiment, the upper and lower edges 108f and 108g may be rounded using a non-uniform radius of curvature around the perimeter of the implant body. In another alternative embodiment, the upper and lower edges 108f and 108g along the anterior side 108b may be rounded with a greater radius than the remainder of the upper and lower edges 108f and 108g, such that a bull nose outer surface (not shown) is created on the anterior side 108b of the implant body. Rounding upper and lower edges 108f and 108g may facilitate easier insertion of the intervertebral implant body 108, for example by minimizing required distraction of the end plates of adjacent vertebral bodies.

In an example embodiment, the intervertebral implant body 108 has a generally wedge-shaped side-view profile. As illustrated in Figure body 1E, this wedge shape is defined by a gradual decrease in the height of the intervertebral implant body 108 (as measured between the upper and lower surfaces 108d and 108e) extending between the posterior side 108a in the direction of the anterior side 108b. The intervertebral implant body 108 has a generally constant height between lateral sides 108c. In alternative embodiments, the intervertebral implant body 108 may have a gradual increase in height followed by a gradual decrease in height extending from one lateral side 108c to the other, and/or may have a generally constant height between the posterior and anterior sides 108a and 108b, or may have convex and/or concave upper and lower surfaces 108d and 108e, thereby defining a gradual increase in height followed by a gradual decrease in height extending from the posterior side 108a to the anterior side 108b and from one lateral side 108c to the other.

A plurality of grooves 112 may be formed within the intervertebral implant body 108 where the upper and lower surfaces 108d and 108e intersect with the anterior side 108b. The groove 112 may be concave and may be configured to align with arcuate grooves, hereinafter also referred to as curved apertures, 128 of the fixation plate 116 when the intervertebral implant body 108 and the fixation plate 116 are in an assembled configuration. In an example embodiment, the grooves 112 may be substantially smooth and devoid of any protrusions. Retaining grooves 114 may be formed within the lateral sides 108c of the intervertebral implant body 108 between the upper and lower surfaces 108d and 108e. The retaining grooves 114 may be configured to releasably engage complementary engaging ribs 120 of the fixation plate 116.

The fixation plate 116 is defined by a generally C-shaped, channel-like body 118 that includes an anterior side 118a with upper and lower sides 118b and 118c opposite each other, and lateral sides 118d extending from opposite sides of the anterior side 118a in a generally perpendicular direction from the anterior side 118a. The anterior, upper, lower, and lateral sides 118a, 118b, 118c, and 118d may form a generally channel-like structure (in essence, a cradle) which may be configured to receive the anterior side 108b and at least a portion of the lateral sides 108c in partial nested engagement. As such, the upper and lower sides 108b and 108c may define gradual increases and/or decreases in height in a posterior direction from the anterior side 118a and/or between the lateral sides 108d, in order to generally conform the fixation plate 116 to the geometry of the intervertebral implant body 108. The lateral sides 118d may have engaging ribs 120 formed thereon at the ends opposite the anterior side 118a, the engaging ribs 120 configured to be releasably received within the retaining grooves 114 of the intervertebral implant body 108.

The anterior side 118a of the fixation plate 116 may have a pair of apertures 122 formed therethrough configured to receive grasping features of a delivery instrument. In an example embodiment, the apertures 122 may be D-shaped, as illustrated in Figure 1C. However any other aperture shape may be defined as appropriate. The apertures 122 may have a retaining rib 124 formed therein configured to engage with a complimentary grasping rib of the delivery instrument. The anterior side 118a of the fixation plate 116 may also have a central bore 126 formed therethrough having an inner surface 126a with threads configured, to engage complimentary threads of a locking screw 138. The anterior side 118a of the fixation plate 116 may also have a concave recess 130 formed therein configured to receive a complimentary convex surface 134d of the blocking plate 132.

The anterior side 118a of the fixation plate 116 may also have a plurality of arcuate grooves 128 formed therethrough configured to slidably receive the arcuate fixation members 12C and to define an insertion trajectory for each of the arcuate fixation members 12C. In an example embodiment, the arcuate grooves 128 may have a generally uniform cross sectional geometry configured to closely conform to the cross sectidnal geometry of the body 102 of the arcuate fixation member 12C between the head 106 and the distal end 102b. When an arcuate fixation member 12C is in a fully inserted position within a respective arcuate groove 128, the lower surface 106b of the head 106 will be engaged with the outer surface of the anterior side 118a of the fixation plate 116. Because the upper surface 106a of the head 106 will not be flush with the outer surface of the anterior side 118a of the fixation plate 116 in this configuration, it may be desirable to omit the blocking plate 132 and the locking screw 138. In an alternative embodiment, the arcuate grooves 128 have a recessed ledge formed therein in the area where the arcuate grooves 128 intersect with the outer surface of the anterior side 118a of the fixation plate 116, the recessed ledge being configured to receive the lower surface 106b of the head 106 when the arcuate fixation member 12C is in a fully inserted position, such that the upper surface 106a of the head 106 is substantially flush with the outer surface of the anterior side 118a of the fixation plate 116.

The arcuate grooves 128 may be disposed about the central bore 126 in any desired configuration and may define any insertion trajectories as appropriate. In the example embodiment depicted in Figures 1C to 1J, the arcuate grooves 128 are formed in opposing quadrants around the central bore 126, with two arcuate grooves 128 located near the upper side 118b and defining two generally cranial insertion trajectories, and two arcuate grooves 128 located near the lower side 118c and defining two generally caudal insertion trajectories. It should be noted that this configuration of arcuate groove 128 locations and arcuate fixation member 12C insertion trajectories is merely an example, and the scope of the instant disclosure should not be limited thereto.

The blocking plate 132 is defined by a generally disc-shaped body 134 with planar upper and lower surfaces 134a and 134b, an anterior surface 134c, and a posterior surface 134d. The upper and lower surfaces 134a and 134b and the height of the body 134 (as measured between the upper and lower surfaces 134a and 134b) may be defined to match the height (as measured between the upper and lower surfaces 118b and 118c) of the anterior side 118a of the fixation plate 116 when the blocking plate 132 is in a fully assembled configuration. The anterior surface 134c of the body 134 may be generally planar, or may be defined to match the outer surface of the anterior side 118a of the fixation plate 116 when the blocking plate 132 is in a fully assembled configuration. The posterior surface 134d may be defined as a convex surface configured to engage with the concave recess 130 formed in the anterior side 118a of the fixation plate 116 when the blocking plate 132 is in a fully assembled configuration. The body 134 may have an aperture 136 formed therethrough. In an example embodiments, the diameter of the aperture may be slightly larger than the diameter of the central bore 126 of the fixation plate 116, such that a locking screw 138 may be inserted into the aperture with no interference therebetween. In another embodiment, the diameter of the aperture 136 may be substantially the same as that of the central bore 126, and the inner surface of the aperture may have threads formed thereon, the threads configured to engage complimentary threads of the locking screw 138. The aperture 136 may further be defined by a concave recess 136a formed within the anterior surface 134c, the concave recess 136a configured to receive the convex head 142 of the locking screw 138.

The locking screw 138 includes a shaft 140 that defines longitudinally opposing proximal and distal ends 140a and 140b, respectively, and a head 142 coupled to the proximal end 140a of the shaft 140, either directly or indirectly via an unthreaded neck 144 that is coupled between the proximal end 140a of the shaft 140 and the head 142. The head 142 can define a generally convex shape between the interface of the head 142 and the neck 144 that extends outward towards a proximal end 142a of the head 142. The convex shape of the head may be configured to engage the concave recess 136a of the blocking plate 132. Of course, the head 142 can assume any other suitable alternative shape as appropriate. Helical threads 146 extend radially out from the shaft 140 at locations at and between the proximal and distal ends 140a and 140b that are configured to engage complementary threads on the inner surface 126a of the central bore 126 of the fixation plate 116. Thus, a substantial entirety of the shaft 140 between the proximal and distal ends 140a and 140b may be threaded. The distal end 142a of the head 142 may have driving features 142b defined therein, designed to engage with complementary driving features of a delivery instrument.

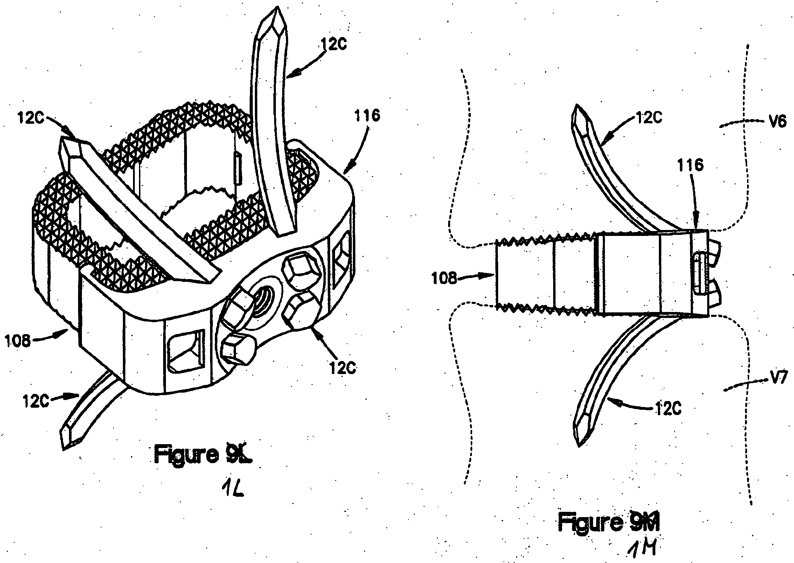

Referring now to Figures 1L and 1M, an example embodiment of the intervertebral implant system 100 in a nearly completely assembled configuration is illustrated. Figure 1L depicts the intervertebral implant system 100 partially assembled outside of an intervertebral space (the blocking plate and locking screw have been omitted for simplicity). The intervertebral implant body 108 has been seated within the fixation plate 116 such that the retaining ribs are seated with the retaining grooves on the lateral sides of the intervertebral implant body 108. Four arcuate fixation members 12C have been inserted through corresponding arcuate grooves within the fixation plate 116, and have been driven to an almost fully inserted position. In a final assembled configuration, the arcuate fixation members 12C would be driven into their fully inserted position, the blocking plate would be received within the concave recess in the anterior side of the fixation plate, and the locking screw would be driven into the central bore of the fixation plate and finally tightened, thereby blocking the arcuate fixation members 12C from backing out of the assembled intervertebral implant system 100.

Figure 1M depicts an example embodiment of the intervertebral implant system 100 partially assembled inside of an intervertebral space between adjacent vertebral bodies V6 and V7 (the blocking plate and locking screw have been omitted for simplicity). As an initial step, the intervertebral implant body 108 has been prepared for insertion, for example by being packed a with bone growth inducing substance and or/having its outer surfaces properly prepared, and has been seated within the fixation plate 116 such the retaining ribs are seated with the retaining grooves on the lateral sides of the intervertebral implant body 108. The intervertebral implant body 108 was then inserted into the intervertebral space between the adjacent vertebral bodies V6 and V7using a delivery instrument that is described in greater detail below. The delivery instrument was then used to deliver the four arcuate fixation members 12C into the arcuate grooves in the fixation plate and drive them into an almost fully inserted position. During the final steps of the assembly process, the delivery instrument would be used to drive the arcuate fixation members 12C into their fully inserted position, the blocking plate would be received within the concave recess in the anterior side of the fixation plate, and the locking screw would be driven into the central bore of the fixation plate and finally lightened, thereby blocking the arcuate fixation members 12C from backing out of the assembled intervertebral implant system 100.

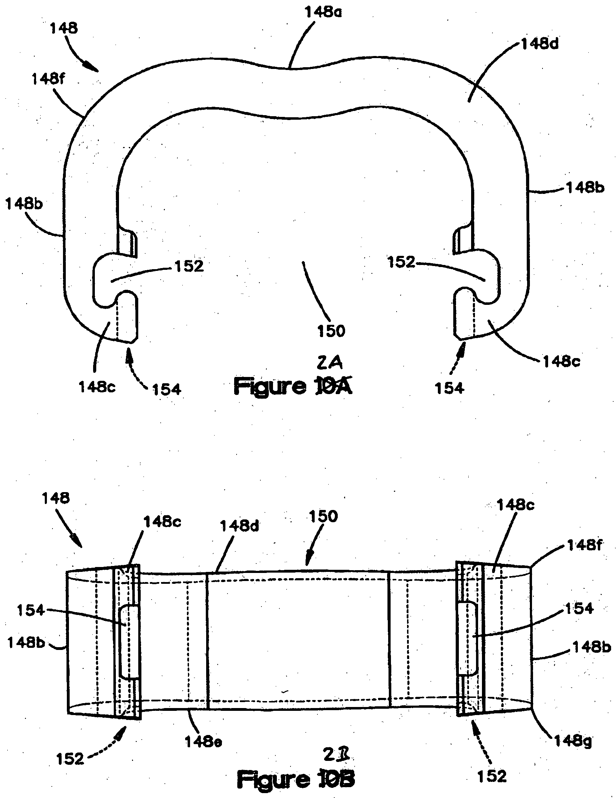

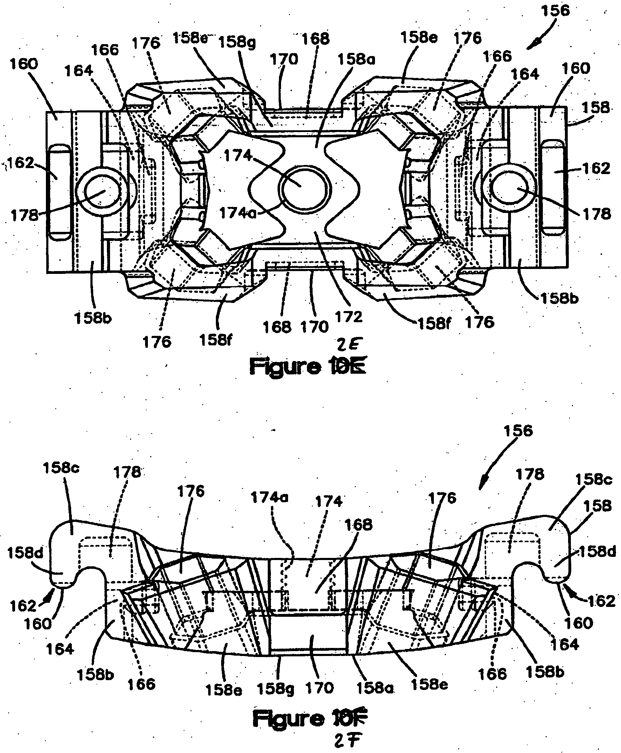

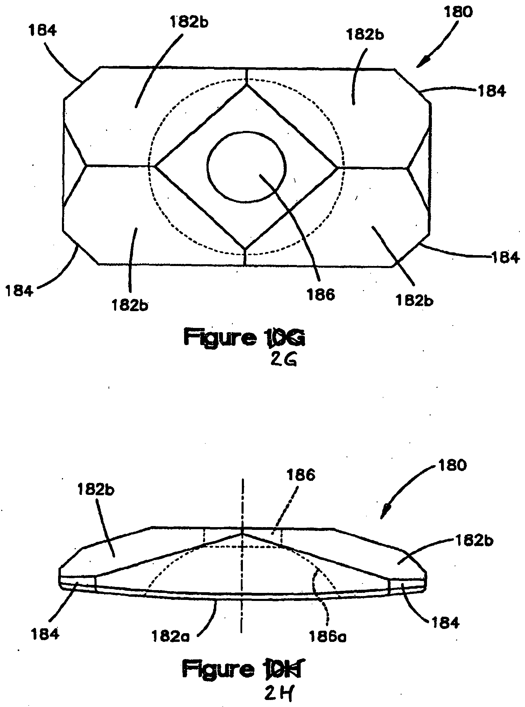

Referring now to Figures 2A to 2J, another example embodiment of the intervertebral implant system 100 comprising an arcuate fixation member 12C, an intervertebral implant body 148, a fixation plate 156, a blocking plate 180, and a locking screw 138 is illustrated.

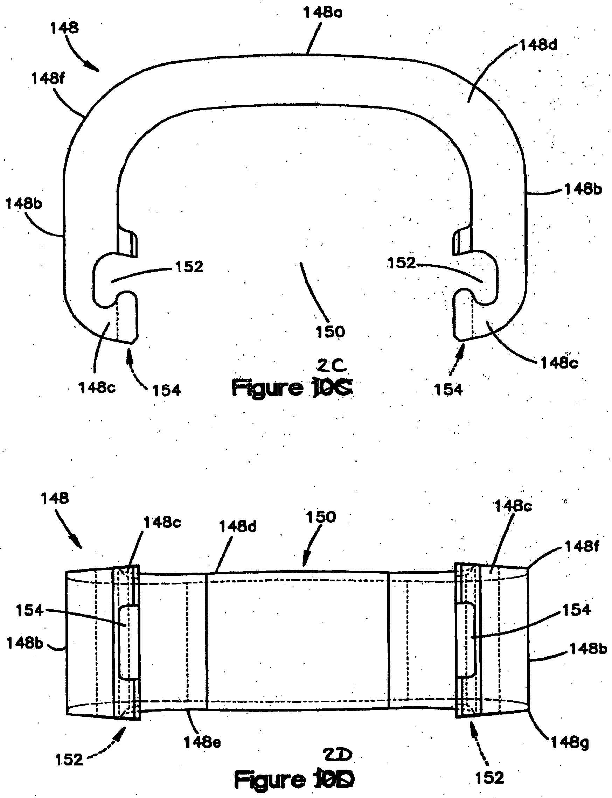

The intervertebral implant body 148 has a generally C-shaped footprint defined by a posterior side 148a, lateral sides 148b terminating in distal ends 148c opposite the posterior side 148a, an upper surface 148d, and a lower surface 148e opposite the upper surface. In an example embodiment, a portion of the posterior side 148a between the lateral sides 148b may be curved inwardly in a direction toward the distal ends 14c, as depicted in Figures 2A and 2B. In an alternative embodiment, a portion of the posterior side 148a between the lateral sides 148b may be curved outwardly in a direction away from the distal ends 148c (not shown). In another alternative embodiment, the posterior side 148a may be substantially straight between the lateral sides 148b, as depicted in Figures 2C and 2D. The posterior side 148a and lateral sides 148b define an open central bore 150, the shape of which substantially conforms to the footprint of the intervertebral implant body 148. The central bore 150 can be filled with bone growth inducing substances to allow bony ingrowth and to assist in fusion between the intervertebral implant body 148 and adjacent vertebral bodies.

In an example embodiment of the intervertebral implant body 148, the upper and lower surfaces 148d and 148e may have gripping features such as teeth, spikes, or similar structures formed thereon and configured to facilitate gripping engagement between the upper and lower surfaces 148d and 148e and the end plates of adjacent vertebral bodies. The teeth may be pyramidal, saw toothed or other similar shapes. In alternative embodiments of the intervertebral implant body 148, portions of and/or the entirety of the upper and lower surfaces 148d and 148e may be substantially smooth and devoid of any protrusions. Upper and lower edges 148f and 148g, defined where the upper and lower surfaces 148d and 148e intersect with the posterior and lateral sides 148a and 148b respectively around the perimeter of the intervertebral implant body 148, may be rounded (not shown) In an example embodiment, the upper and lower edges 148f and 148g may be rounded using a uniform radius of curvature around the perimeter of the implant. In an alternative embodiment, the upper and lower edges 148f and 148g may be rounded using a non-uniform radius of curvature around the perimeter of the implant body. Rounding upper and lower edges 148f and 148g may facilitate easier insertion of the intervertebral implant body 148, for example by minimizing required distraction of the end plates of adjacent vertebral bodies.

In an example embodiment, the intervertebral implant body 148 has a generally wedge-shaped side-view profile. This wedge shape is defined by a gradual increase in the height of the intervertebral implant body 148 (as measured between the upper and lower surfaces 148d and 148e) extending outwardly in a direction away the posterior side 148a in the direction of the distal ends 148c. The intervertebral implant body 148 has a generally constant height between lateral sides 148b. In alternative embodiments, the intervertebral implant body 148 may have a gradual increase in height followed by a gradual decrease in height extending from one lateral side 148b to the other, and/or may have a generally constant height between the posterior sides 148a and the distal ends 148c, or may have convex and/or concave upper and lower surfaces 148d and 148e, thereby defining a gradual increase in height followed by a gradual decrease in height extending from the posterior side 148a to the distal ends 148c and from one lateral side 148b to the other.

Retaining grooves 152 may be formed within the distal ends 148c of the intervertebral implant body 148, for example in a vertical direction substantially perpendicular to a horizontal midplane defined between the upper and lower surfaces 148d and 148e. The retaining grooves 152 may be configured to releasably engage complementary retaining ribs 160 of the fixation plate 156. The distal ends 148c may also have access grooves 154 formed therein between the upper and lower surfaces 148d and 148e. The access grooves 154 may be configured to align with complimentary access grooves 162 and/or 164 of the fixation plate 156, thereby defining an access cavity 168 for an engaging member of a delivery instrument when the intervertebral implant body 148 and the fixation plate 156 are in an assembled configuration.

The fixation plate 156 is defined by a generally rectangular body 158 that includes an anterior side 158a and lateral sides 158b extending therefrom, the lateral sides 158b configured to engage with the retaining grooves 152 of the intervertebral implant body 148. In an example embodiment, the lateral sided 158b are generally J-shaped, extending initially from opposite sides of the anterior side 158a in a direction perpendicular to and away from the anterior side 158a, and through curved sections 158c before returning in a direction perpendicular to and towards the anterior side 158a and terminating in distal ends 158d. It should be noted that this configuration for lateral sides 158b is merely an example, and any other geometry may be used as appropriate. Upper and lower edges of the anterior side 158a, defined where upper and lower surfaces 158e and 158f of the anterior side intersect with an anterior surface 158g of the anterior side, may be rounded (not shown). In an example embodiment, the upper and lower edges 158e and 158f may be rounded using a uniform radius of curvature. In an alternative embodiment, the upper and lower edges 158e and 158f may be rounded using a non-uniform radius of curvature. Rounding upper and lower edges 158e and 158f may facilitate easier insertion of the fixation plate 156, for example by minimizing required distraction of the end plates of adjacent vertebral bodies.

The lateral sides 158b may have retaining ribs 160 formed thereon at the distal ends 158d, the retaining ribs 160 configured to be releasably received within the retaining grooves 152 of the intervertebral implant body 148. Access grooves 162 and 164 may be formed within the retaining ribs 160 and the lateral sides 158b, in the area where the lateral sides 158b interface with the anterior side 158a, respectively. The access grooves 162 and 164 may be configured to align with complimentary access grooves 154 of the intervertebral implan body 148, thereby defining an access cavity 168 for receiving an engaging feature of a delivery instrument when the intervertebral implant body 148 and the fixation plate 156 are in an assembled configuration. The access grooves 164 may have a retaining shelf 166 formed therein configured to engage with an engaging feature of a delivery, instrument, for example the raised ribs 258d formed on the insertion rods, hereinafter also referred to a lateral areas, 258 of the delivery instrument 278, described in greater detail below. The lateral sides 158b may also have bores 178 formed within the curved sections 158c, the apertures configured to receive, for example the distal engagement tips 258c of the insertion rods of 258 of the delivery instrument 278.

The anterior side 158a of the fixation plate 156 may have gripping grooves 168 formed within the upper and lower surfaces 158e and 158f of the anterior side 158a, the gripping grooves 168 configured to receive grasping arms of a delivery instrument. The gripping grooves 168 may have a gripping ridge 170 formed therein, the gripping ridge configured to be engaged by the complimentary grasping features formed at the ends of the grasping arms of the delivery instrument. The anterior side 158a of the fixation plate 156 may also have a recess 172 formed therein configured to receive additional components of the intervertebral implant systems 100, for example a ratchet blade 188, a blocking plate 180, or the like. The anterior side 158a may also have a central bore 174 formed therethrough having an inner surface 174a with threads configured to engage complimentary threads of a locking screw 138. In an example embodiment, the central bore 174 may be formed within the recess 172.

The anterior side 158a of the fixation plate 156 may also have a plurality of arcuate grooves 176 formed therethrough configured to slidably receive the arcuate fixation members 12C and to define an insertion trajectory for each of the arcuate fixation members 12C. In an example embodiment, the arcuate grooves 176 may have a generally uniform cross sectional geometry configured to closely conform to the cross sectional geometry of the body 102 of the arcuate fixation member 12C between the head 106 and the distal end 102b. When an arcuate fixation member 12C is in a fully inserted position within a respective arcuate groove 176, the lower surface 106b of the head 106 will be engaged with the outer surface of the anterior side 158a of the fixation plate 156. Because the upper surface 106a of the head 106 will not be flush with the outer surface of the anterior side 158a of the fixation plate 156 in this configuration, it may be desirable to omit the blocking plate 180 and the locking screw 138. In an alternative embodiment, the arcuate grooves 176 have a recessed ledge formed therein in the area where the arcuate grooves 176 intersect with the outer surface of the anterior side 158a of the fixation plate 156, the recessed ledge being configured to receive the lower surface 106b of the head 106 when the arcuate fixation member 12C is in a fully inserted position, such that the upper surface 106a of the head 106 is substantially flush with the outer surface of the anterior side 158a of the fixation plate 156.

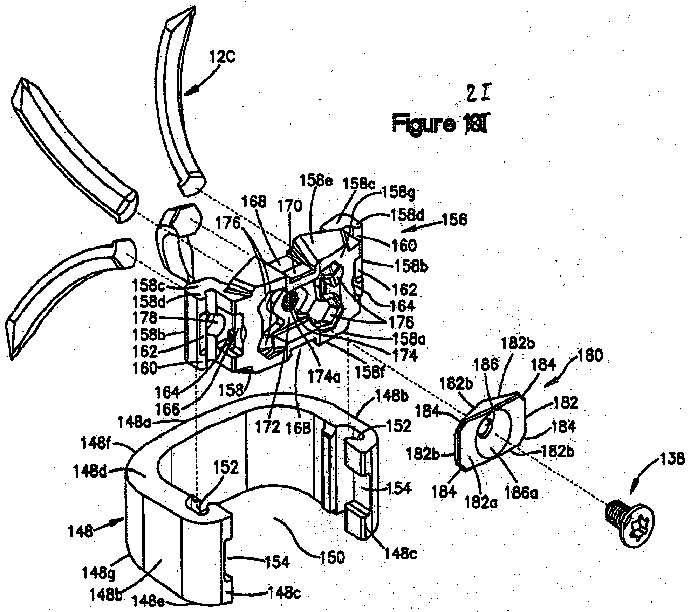

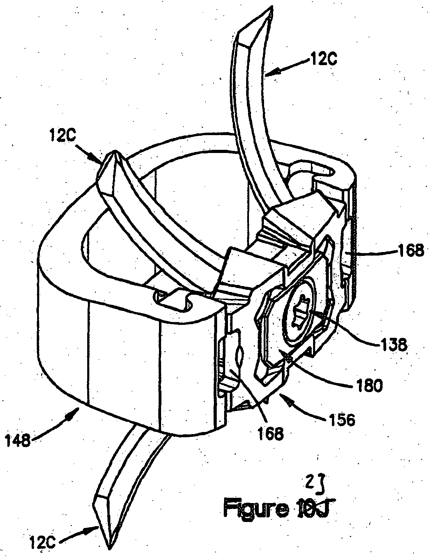

The arcuate grooves 176 may be disposed about the central bore 174 in any desired configuration and may define any insertion trajectories as appropriate. In the example embodiment depicted in Figures 2E, 2E, 2I and 2J, the arcuate grooves 176 are formed in opposing quadrants around the central bore 174, with two arcuate grooves 176 located near the upper surface 158e and defining two generally cranial insertion trajectories, and two arcuate grooves 176 located near the lower surface 158f and defining two generally caudal insertion trajectories. It should be noted that this configuration of arcuate groove 176 locations and arcuate fixation member 12C insertion trajectories is merely an example, and the scope of the instant disclosure should not be limited thereto.

The blocking plate 180 is defined by a generally rectangular body 182 with an anterior surface 182a, and a plurality of angled posterior surfaces 182b generally opposite the anterior surface 182a. The body 182 may have an aperture 186 formed therethrough In an example embodiment, the diameter of the aperture may be slightly larger than the diameter of the central bore 174 of the fixation plate 156, such that a locking screw 138 may be inserted into the aperture with no interference therebetween. In another embodiment, the diameter of the aperture 186 may be substantially the same as that of the central bore 174, and the inner surface of the aperture may have threads formed thereon, the threads configured to engage complimentary threads of the locking screw 138. The aperture 186 may further be defined by a concave recess 186a formed within the anterior surface 182a, the concave recess 186a configured to receive the convex head 142 of the locking screw 138. The height, width, and depth of the body 182 may be proportioned so that the blocking plate 180 will be received within the recess 172 of the fixation plate 156, such that the anterior surface 182a of the body 182 is substantially flush with the anterior surface 158g of the anterior side 158a of the fixation plate 156 when the fixation plate 156 and the blocking plate 180 are in an assembled configuration. -The anterior surface 182a of the body 182 may be generally planar, or may be defined to match the outer surface of the anterior side 158a of the fixation plate 156 when the blocking plate 180 and the fixation plate 156 are in a fully assembled configuration.

In an example embodiment wherein the blocking plate 180 and locking screw 138 are installed after the intervertebral implant 148 and fixation plate 156 have been inserted into an intervertebral space and the arcuate fixation members 12C driven into their fully inserted positions, the angled posterior surfaces 182b and chamfered corners 184 of the blocking plate 180 may be configured to engage the heads 106 of the arcuate fixation members 12C within the recess 172 of the fixation plate 156 when the blocking plate 180 is installed followed by the locking screw 138. When final tightening of the locking screw 138 is performed, the blocking plate 180 may rigidly fix the arcuate fixation members 12C in position, and additionally prevent pullout of the arcuate fixation members 12C.

In another example embodiment wherein the intervertebral implant body 148, the fixation plate 156, the blocking plate 180, and the locking screw 138 are pre-assembled, but not finally tightened, and then inserted into an intervertebral space before the arcuate fixation members 12C are inserted and driven into position, the angled posterior surfaces 182b and chamfered corners 184 of the blocking plate 180 may be configured to allow the arcuate fixation members 12C to be inserted and driven into position with the blocking plate 180 and the locking screw 138 in place. In this embodiment, the angled posterior surfaces 182b may have wedge features formed thereon (not shown), the wedge features configured to interfere between the heads 106 of the arcuate fixation members and the surrounding structure of the fixation plate 156, for example by applying outward force laterally upward and downward on the arcuate fixation members 12C to lock them in place when final tightening is applied to the locking screw, and additionally to prevent pullout of the arcuate fixation members 12C.

Referring now to Figure 2J, an example embodiment of the intervertebral implant system 100 in a completely assembled configuration outside of an intervertebral space is illustrated. The fixation plate 156 has been engaged with the intervertebral implant body 148 such that the retaining ribs of the fixation plate 156 are seated with the retaining grooves of the intervertebral implant body 148. Four arcuate fixation members 12C have been inserted through corresponding arcuate grooves within the fixation plate 156, and have been driven to a fully inserted position. The blocking plate 180 and the locking screw 138 have been installed and finally tightened.

It should be noted that although the description and accompanying figures illustrating the intervertebral implant system 100 included herein depict example embodiments of the intervertebral implant system 100 that include four arcuate fixation members 12C, with two of the four arcuate fixation members 12C having a generally cranial insertion trajectory and the remaining two arcuate fixation members having a generally caudal insertion trajectory, other configurations of the intervertebral implant system 100 using more or less arcuate fixation members 12C and/or varying insertion trajectories are possible and intended to be included within the scope of the instant disclosure. For example, in an alternative embodiment of the intervertebral implant system 100, the fixation plate 116 may have three arcuate grooves 128 formed therein having any desirable placement and/or insertion trajectory with respect to the central bore 126 (*e*.*g*., with two of the three arcuate fixation members 12C having a generally caudal insertion trajectory and the remaining arcuate fixation member 12C having a generally cranial insertion trajectory, or with two of the three arcuate fixation members 12C having a generally cranial insertion trajectory and the remaining arcuate fixation member 12C having a generally caudal insertion trajectory). The intervertebral implant body 108 may of course have matching grooves 112 formed therein. Such alternative embodiments with two arcuate fixation members 12C having one of a generally cranial or caudal trajectory and a third arcuate fixation member having the opposite general trajectory may allow for the stacking of two or more assembled configurations of the intervertebral implant system 100 in place of adjacent vertebral bodies removed from an intervertebral space. Additionally, while the arcuate fixation members 12C illustrated in the various figures herein generally have divergent insertion trajectories with respect to each other, fixation plates 116 and/or 156 may also be configured so that one or more of the arcuate fixation members 12C will have convergent insertion trajectories with respect to each other, or similar insertion trajectories (e.g., laterally towards a common side). For example, arcuate fixation members with generally cranial insertion trajectories may converge toward one another, may diverge away from one another, or may both follow similar insertion trajectories, while arcuate fixation members with generally caudal insertion trajectories may converge toward one another, may diverge away from one another, or may both follow similar insertion trajectories. Any combination of the above insertion trajectory configurations may be used as appropriate.

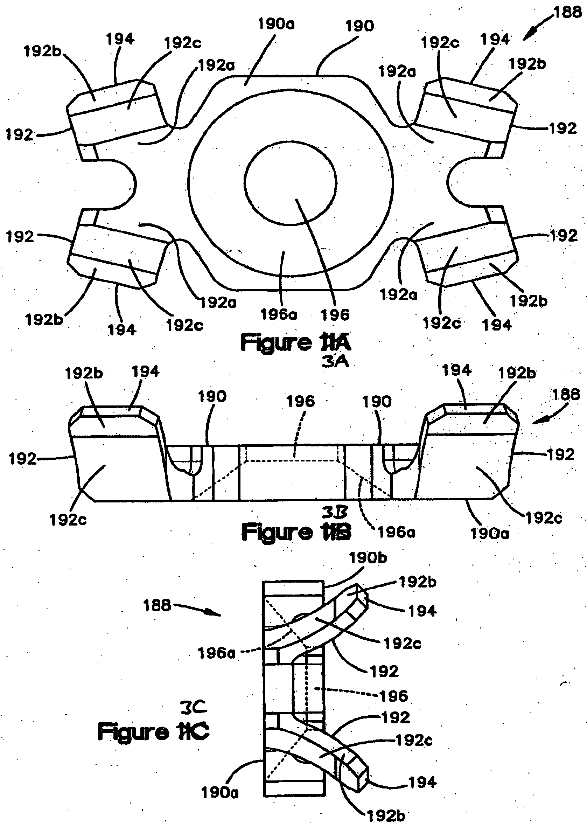

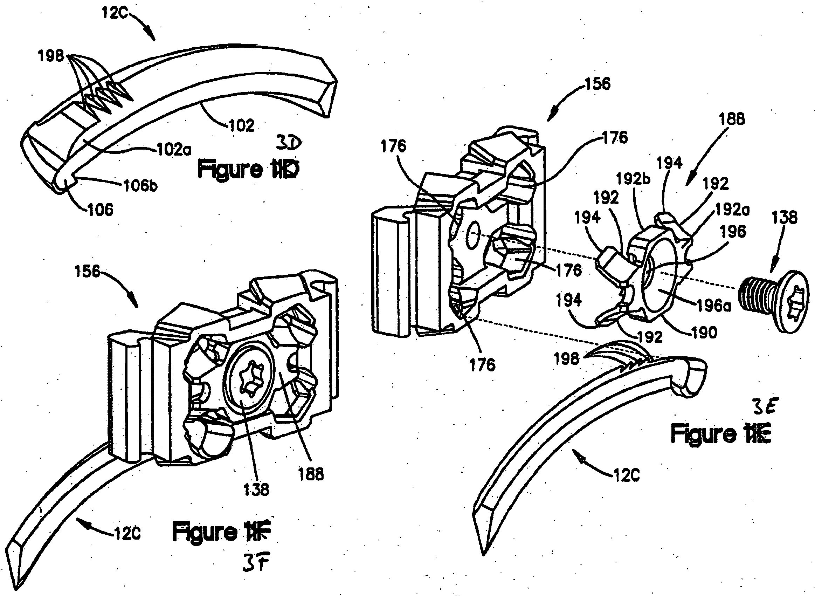

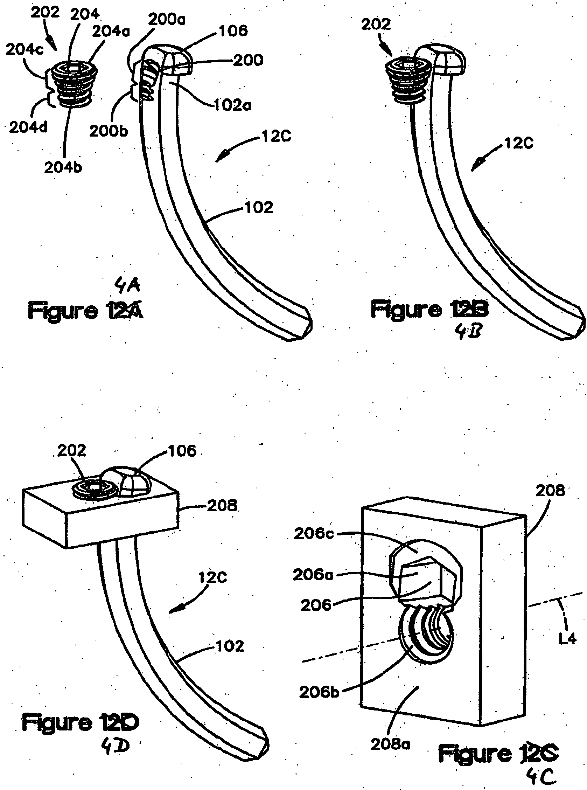

Referring now to Figures 3A to 3H, and Figures 4A to 4C, example embodiments of additional features for securing the arcuate fixation members 12C within assembled configurations of the intervertebral implant system 100 are illustrated. Referring first to Figures 3A to 3H, an example embodiment using ratcheting features to secure the arcuate fixation members 12C is illustrated. In an example embodiment, an intervertebral implant body 148 (not shown), a fixation plate 156, and a locking screw 138 can be used in combination with a ratchet plate 188 configured to be received within the fixation plate 156 and fixed in place with the locking screw 138. The ratchet plate 188 is defined by a body 190 defining an anterior surface 190a and a posterior surface 190b opposite the anterior surface.

The body 190 includes a plurality of blades 192 extending outwardly therefrom, each blade 192 defining a proximal end 192a where the blade 192 extends from the body 190, and a distal end 192b opposite the proximal end 192a. In an example embodiment, the blades 192 may be curved between the proximal and distal ends 192a and 192b. The blades 192 may be curved along a generally constant radius of curvature, may be curved along a non-uniform radius of curvature, or any combination thereof. In an alternative embodiment, the blades may be substantially straight between the proximal and distal ends 192a and 192b. The blades may configured to be of a length such that the distal ends 192b of the blades 192 are disposed within the arcuate grooves 176 of the fixation plate 156 when the ratchet plate 188 is inserted into the recess 172 of the fixation plate 156 in an assembled configuration. It should be noted that while the ratchet plate 188 is depicted within the attached figures as having four blades 192, this number of blades 192 is only an example configuration, and the scope of the instant disclosure should not be limited thereto. For example, alternative embodiments of the ratchet blade having more or less than four blades 192 are possible and intended to be included within the scope of the instant disclosure.

The distal ends 192b of the blades 192 may have tips 194 defined thereon, the tips 194 configured to lockingly engage with a complimentary engaging feature, for example a ratchet tooth 198, defined on an arcuate fixation member 12C. The body 190 may have an aperture 196 formed therethrough. In an example embodiment, the diameter of the aperture may be slightly larger than the diameter of the central bore 174 of the fixation plate 156, such that a locking screw 138 may be inserted into the aperture with no interference therebetween. In another embodiment, the diameter of the aperture 196 may be substantially the same as that of the central bore 174, and the inner surface of the aperture may have threads formed thereon, the threads configured to engage complimentary threads of the locking screw 138. The aperture 196 may further be defined by a concave recess 196a formed within the anterior surface 190a, the concave recess 196a configured to receive the convex head 142 of the locking screw 138.

Referring now to Figure 3D, an example embodiment of an arcuate fixation member 12C configured for use with the ratchet plate 188 is illustrated. A series of ratchet teeth 198 are defined on the body 102 of the arcuate fixation member 12C. The ratchet teeth 198 may be defined in the area of the proximal end 102a of the body, near the head 106. The ratchet teeth 198 may be formed from triangular grooves formed within the body 102 of the arcuate fixation member, or may be formed on or in the body 102 using any other tooth geometry as appropriate. It should be noted that while the arcuate fixation member 12C depicted in Figures 3D to 3H has four ratchet teeth 198 formed thereon, this number of ratchet teeth is only an example, and the scope of the instant disclosure should not be limited thereto.

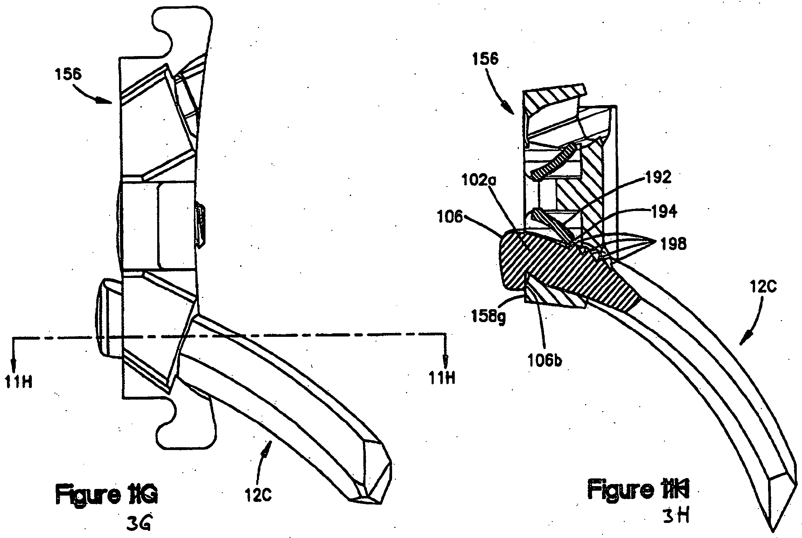

Referring now to Figures 3F to 3H, an example embodiment of the intervertebral implant system 100 in a partially assembled configuration including a single arcuate fixation member 12C and omitting the intervertebral implant 148 is illustrated. The ratchet plate 188 has been nested within the fixation plate 156 and anchored in place with the locking screw 138. A single arcuate fixation member 12C has been inserted through a corresponding arcuate groove within the fixation plate 156, and has been driven to a fully inserted and locked position. Figures 3G and 3H illustrate a cross sectional view of the partially assembled intervertebral implant system 100. The arcuate fixation member 12C has been driven into place such that each successive ratchet tooth 198 has passed over the tip 194 of the blade 192 until the arcuate fixation member 12C arrived in a fully inserted and locked position. The tip 194 of the blade 192 is firmly seated against the ratchet tooth 198 nearest the proximal end 102a of the body 102 of the arcuate fixation member 12C, thereby engaging the lower surface 106b of the head 106 of the arcuate fixation member 12C against the anterior surface 158g of the fixation plate 156 in a fixed and locked configuration.

Referring now to Figures 4A to 4D, an example embodiment for securing an arcuate fixation member 12C of the intervertebral implant system 100 with a screw 202 is illustrated. A plurality of helical threads 200 are defined within the body 102 of the arcuate fixation member 12C, the threads 200 configured to engage complimentary threads of the screw 202. The threads 200 may be defined within the area of the proximal end 102a of the body 102, near the head 106. In an example embodiment, the threads 200 may define tapered threads 200a that transition into constant radius threads 200b. The radius of the tapered threads 200a may decrease as the tapered threads 200a extend in a direction downward and perpendicular from the proximal end 102a of the body 102 until the tapered threads 200a transition into the constant radius threads 200b.

The screw 202 includes a shaft 204 that defines longitudinally opposing proximal and distal ends 204a and 204b, respectively. A plurality of helical tapered threads 204c may extend along the shaft 204 in a direction toward the distal end 204b. The outer diameter of the tapered threads 204c may decrease with distance from the proximal end 204a until the tapered threads 204c transition into a series of constant radius threads 204d. The constant radius threads 204d may extend along the remainder of the shaft 204 to the distal end 204b. The tapered and constant radius threads 204c and 204d may be configured to be complimentary to the tapered and constant radius threads 200a and 200b of the arcuate fixation member 12C. In an alternative embodiment, the threads 200 defined within the body 102 and the threads defined on the shaft 204 of the screw 202 may be of constant radius. It should be noted that although the screw 202 is depicted in Figures 4A to 4D as a set-type screw, that any appropriate screw-type anchor may be utilized.

When deployed in combination with the fixation plate 156, one or more of the arcuate grooves 176 may be replaced with a threaded arcuate groove 206. For the sake of simplicity, the threaded arcuate groove 206 is illustrated in Figures 4C and 4D as defined within block 208, rather than in the fixation plate 156. The threaded arcuate groove 206 includes an arcuate groove 206a configured to receive the arcuate fixation member 12C, and a threaded aperture 206b formed adjacent to the arcuate groove 206a and configured to guide a screw type fastener along a longitudinal axis L4. It should be noted that although the threaded aperture 206b is depicted as being formed to define the longitudinal axis L4 in a direction generally perpendicular to an outer surface a of the block 208, the threaded aperture 206b may be formed to define the longitudinal axis L4 in any direction appropriate for insertion of a screw type fastener. A head recess 206c may be formed within the threaded arcuate groove 206 in the area where the threaded arcuate groove 206 meets the outer surface 208a of the block 208, the head recess 206c configured to receive at least a portion of the head 106 of the arcuate fixation member 12C.

In the assembled configuration depicted in Figure 4D, the arcuate fixation member 12C has been inserted into the arcuate groove 206a and driven to a fully inverted position. The screw 202 has been driven into an almost fully driven position in the threaded aperture 206b. As the screw 202 is finally tightened, the tapered threads 204c will engage the complimentary tapered threads 200a of the arcuate fixation member 12C, causing the arcuate fixation member 12C to be locked into its fully inserted position as the screw 202 is tightened. In the finally tightened position, the engagement of the tapered threads 204c, of the screw 202 with-the arcuate fixation member 12C and the threaded aperture 206b will secure the assembly in place and prevent pullout of the arcuate fixation member 12C. In an alternative embodiment, the arcuate fixation member 12C is driven to an almost fully inserted position, and as the screw 202 is finally tightened, the tapered threads 204c will engage the complimentary tapered threads 200a of the arcuate fixation member 12C, causing the arcuate fixation member 12C to be driven into its fully inserted position and locked with the screw and the threaded aperture 206b. In example embodiments utilizing a bone screw (not shown) in combination with the arcuate fixation member 12C, the bone screw may be inserted, for example along a trajectory that varies from that of the insertion trajectory of the arcuate fixation member 12C, such that as the bone screw is driven into its fully driven position within the underlying structure or bone, tapered threads on the bone screw interface with complimentary tapered threads 200a of the arcuate fixation member 12C and the threaded aperture 206b, thereby locking the arcuate fixation member 12C and the bone screw together in their finally assembled position within the threaded aperture 206b.

In another alternative embodiment (not shown) for securing the arcuate fixation members 12C within the intervertebral implant system 100, a captive screw is secured within the central bores 126 or 174 of respective fixation plates 116 Or 156. The captive screw is configured to be retained within its respective central bore, but be freely rotatable therein, such that the end of the captive screw nearest the outermost anterior surface of the respective fixation plate is exposed. The captive screw has engagement features formed within its exposed end for engaging a delivery instrument, and helical threads formed radially along its outer surface, the helical threads configured to engage with a plurality of complimentary helical threads 200 defined within the body 102 of the arcuate fixation member 12C along a substantial length of the body 102 between its proximal and distal ends 102a and 102b respectively. An arcuate fixation member 12C may be loaded tip first into an arcuate groove 128 or 176 of the respective fixation plates 116 or 156, such that the threads 200 near the distal end 102b of the body 102 of the arcuate fixation member engage with the helical threads formed on the outer surface of the captive screw. As rotational force is applied to the captive screw by a delivery instrument, the force is transferred to the arcuate fixation member 12C via the engaged complimentary threads, causing the arcuate fixation member to be driven into underlying structure or bone. A plurality of arcuate fixation members may be so loaded into assemblies comprising respective intervertebral implant bodies and fixation plates before those assemblies are inserted into an intervertebral space. The assemblies may then be guided into position in an intervertebral space by a delivery instrument. Once in position, the delivery instrument may engage the captive screw and apply a rotational force thereto, thereby driving the plurality of arcuate fixation members into fully inserted positions within adjacent vertebral bodies. The captive screw may also lock the arcuate fixation members 12C into position within the respective fixation plates during final tightening of the captive screw.

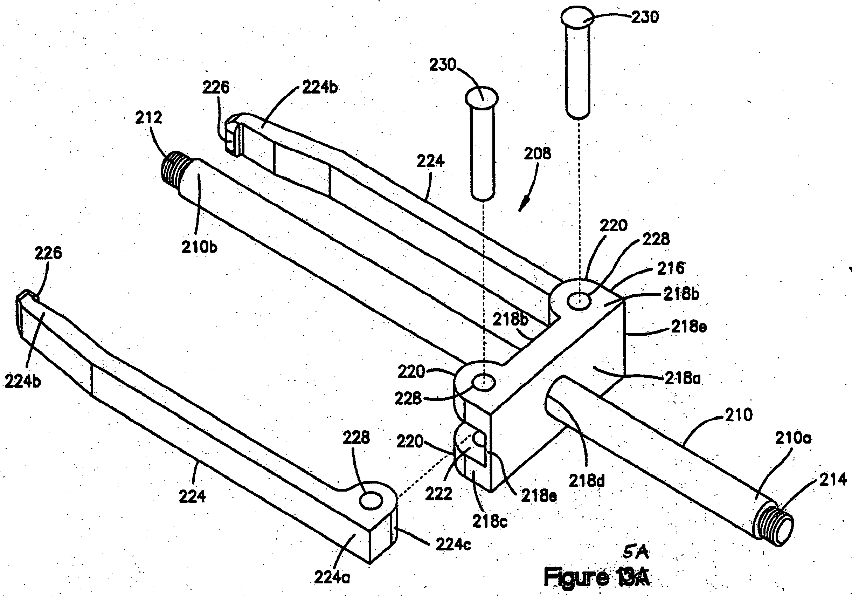

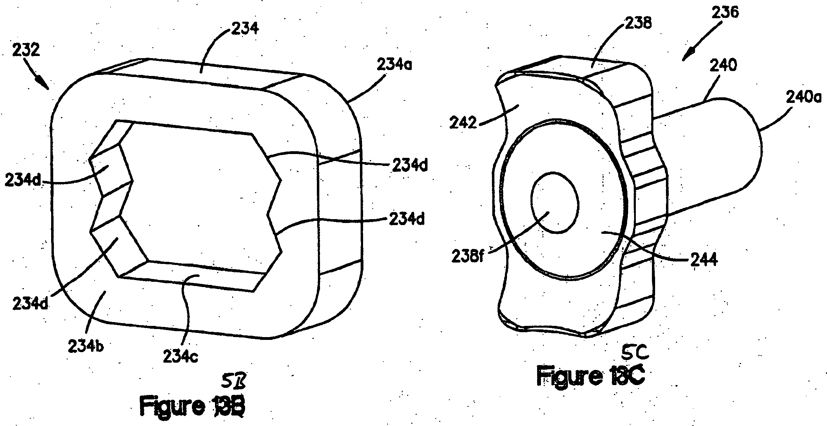

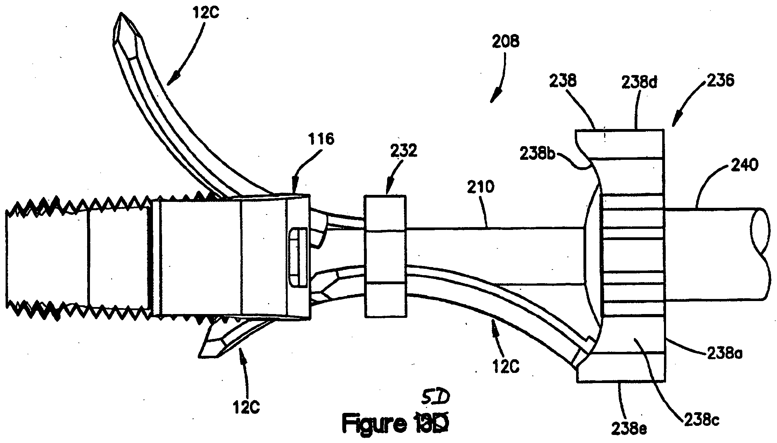

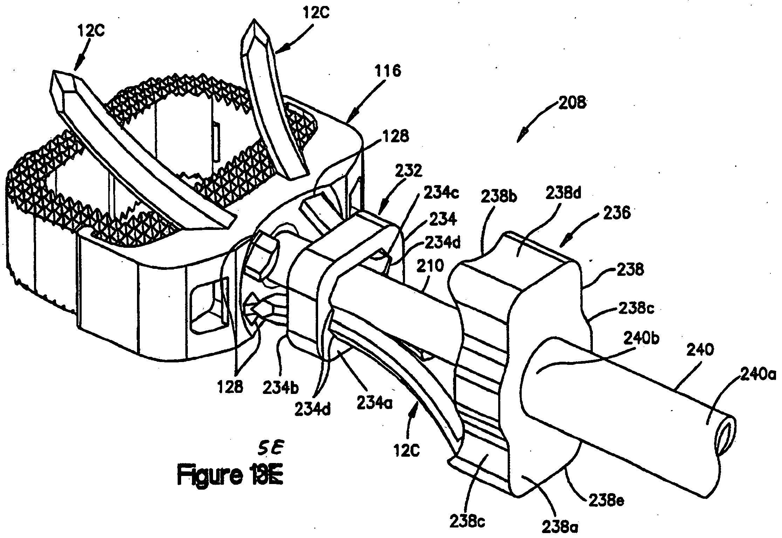

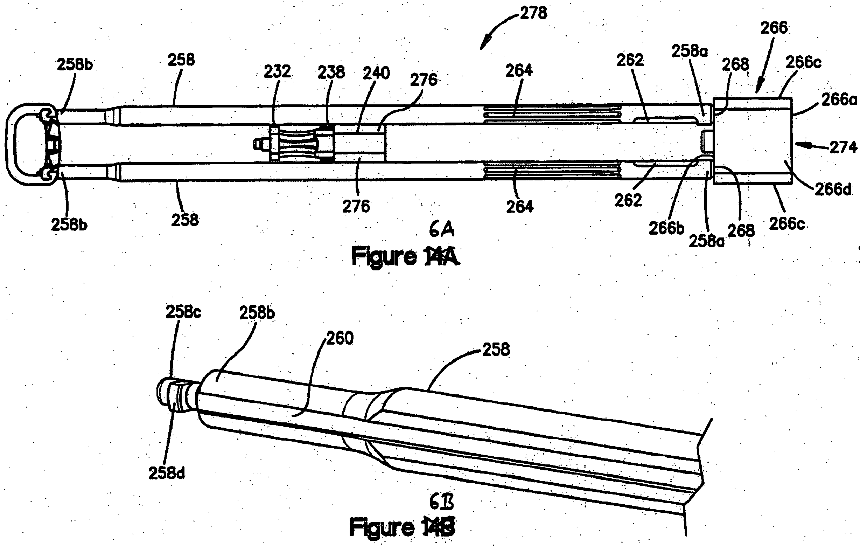

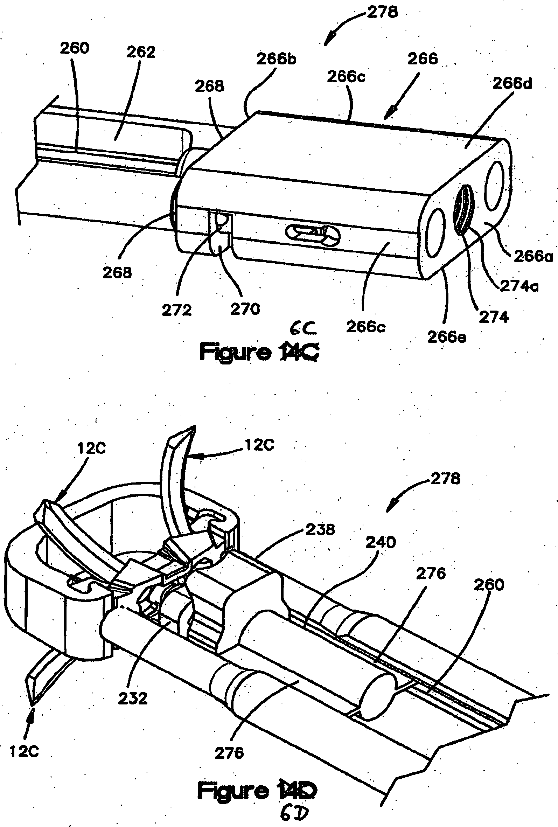

Now referring to Figures 5A to 5F and 6A to 6E, example embodiments of delivery instruments for use in inserting intervertebral implant assemblies constructed using components of the intervertebral implant system 100 into intervertebral spaces and /or driving the arcuate fixation members 12C of the intervertebral implant system 100 are illustrated.

As illustrated in Figures 5A to 5F, delivery instrument 208 includes a drive shaft 210 that defines longitudinally opposing proximal and distal ends 210a and 210b. The distal end 210b of the drive shaft 210 may have helical threads 212 formed thereon, the threads 212 configured to be received in the central bore 126 of the fixation plate 116. The proximal end 210a may have helical threads 214 formed thereon, the threads 214 configured to engage complimentary threads of an accessory (not shown). Accessories may be configured to facilitate easier use of the delivery instrument 208 in positioning and/or insertion of an intervertebral implant assembly into an intervertebral space, and may include a "pistol" grip, a handle, a slap hammer, a slide hammer, or the like. In an alternative embodiment, the proximale end 210a of the drive shaft 210 may have gripping features (not shown) formed directly thereon, the gripping features configured to enable the proximal end 210a of the drive shaft 210 to act as a handle.

Delivery instrument 208 may further include a pivot block 216 having a generally channel-shaped body 218 defined by an anterior side 218a, an upper side 218b, and a lower side 218c opposite the upper side. The anterior side 218a of the body 218 may have an aperture 218d formed therethrough, the aperture enabling the pivot block 216 to be carried on the drive shaft 210. In an example embodiment, the pivot block 216 may be fixed in position on the drive shaft 210. In alternative embodiments, the pivot block 216 may be free to rotate about a longitudinal axis of the drive shaft, free to translate in an anterior and/or posterior direction along the drive shaft 210, may be releasably lockable to the drive shaft 210 between the proximal and distal ends 210a and 210b, or any combination thereof. The upper and lower sides 218b and 218c may define a channel 222 through the body between the opposing ends 218e of the body 218. The upper and lower sides 218b and 218c may each have a pair of raised tabs 220 formed thereon at opposing ends 218e of the body, such that the tabs on the upper and lower sides 218b and 218c directly oppose each other, and are configured to receive a pair of lateral arms 224, one at each of the opposing ends 218e of the body 218.

The lateral arms 224 each include a proximal end 224a and a distal end 224b opposite the proximal end. The distal ends 224b of each of the lateral arms may have an engagement feature 226 formed thereon, the engagement feature 226 configured to engage a complementary engagement feature formed within the fixation plate 116. In an example embodiment, the distal end 224b of each lateral arm 224 may have a raised rib engagement feature 226 formed thereon, the raised rib configured to be inserted into a respective aperture 122 of the fixation plate 116, and to releasably grip a corresponding retaining rib 124. It should be noted that a raised rib is merely an example engagement feature 226, and the scope of the instant disclosure should not be limited thereto. The proximal ends 224a of each of the lateral arms 224 may be configured to be received within the tabs 220 at the opposing ends 218e of the body 218. The tabs 220 and the proximal ends 224a of each of the lateral arms 224 may have apertures 228 formed therethrough, the apertures 228 configured to receive a joint connector 230. The joint connector 230 may be a pin, an axle, a rivet, or any other appropriate connector.

With the proximal ends 224a of each of the lateral arms 224 seated between respective opposing tabs 220 and with the joint connectors 230 inserted into the apertures 228, the lateral arms 224 become configured to pivot about the joint connectors 230, allowing the distal ends 224b of the lateral arms to move in a grabbing motion, such that the distal ends 224b can releasably grip the fixation plate 116. The lateral arms 224 may be configured to be simultaneously pivoted, for example through attachment to a common actuator (not shown). The proximal ends 224a of the lateral arms may be configured with locking features configured to engage with complimentary features of the pivot block 216, to enable the lateral arms 224 to be releasably locked in a gripping position For example, the locking features may comprise a series of ratchet teeth may be formed on rounded end surface 224c of each of the lateral arms, the ratchet teeth configured to engage with a complimentary ratcheting mechanism of the pivot block 216.

Delivery instrument 208 may further include a guide ring 232 and/or a drive head 236 configured to deliver one or more arcuate fixation members 12C for insertion. The guide ring 232 and/or the drive head 236 may also be configured to be carried on the shaft drive 210, preferably between the distal end 210b of the drive shaft 210 and the pivot block 216. The guide ring 232 includes an annular body 234 defining a posterior surface 234a and an anterior surface 234b opposite the posterior surface. The annular body 234 may have a plurality of arcuate grooves, hereinafter also referred to as curved guiding slots, 234d formed along an inner surface 234c of the annular body 234, the arcuate grooves 234d configured to receive a plurality of arcuate fixation members 12C and to align the arcuate fixation members with corresponding arcuate grooves of a fixation plate.

The drive head 236 includes a body 238 defined by an anterior surface 238a, a posterior surface 238b opposite the anterior surface, lateral surfaces 238c, and upper and lower surfaces 238d and 238e respectively. The body 238 may have a central bore 238f formed therethrough, such that the body 238 may be slidably carried on the drive shaft 210. The drive head 236 may further include a shaft 240 having longitudinally defined proximal and distal ends 240a and 240b respectively. The entirety of the shaft 240 may be cannulated, the cannulation defining an inner diameter substantially matching the diameter of the central bore 238f, such that the shaft 240 may be slidably carried on the drive shaft 210. The distal end 240b of the shaft 240 may be coupled to the anterior surface 238a of the body 238.

The posterior surface 238b of the drive head may have a substantially concave outer ring 242 formed therein, the concave outer ring encompassing a substantially convex inner dome 244. The concave outer ring 242 and the convex inner dome 244 are configured to drive the plurality of arcuate fixation members 12C as the drive head 236 travels along the drive shaft 210 in a direction toward the distal end 210b. In an example pre-insertion configuration, the plurality of arcuate fixation members 12C may be loaded into the guide ring 232 and the drive head 236 positioned such that the concave outer ring 242 is engaged with the heads 206 of the arcuate fixation members 12C. As the drive head 236 is advanced towards the distal end 210b of the drive shaft 210, the arcuate fixation members 12C are driven into respective vertebral bodies. As the arcuate fixation members 12C are driven, the heads 106 of the arcuate fixation members 12C travel inwardly along the concave outer ring 242 in a direction generally towards the shaft 210, and from the concave outer ring 242 onto the convex inner dome 244. The convex inner dome 244 may deliver the arcuate fixation members 12C into their fully inserted positions. The guide ring 232 may be removed from the shaft 210 for the final portion of the driving process. It should be noted that while the example drive head 236 depicted in Figures 5C to 5E and described herein is configured to simultaneously drive four arcuate fixation members 12C, other configurations are possible and intended to be within the scope of the instant disclosure. For example, by adjusting the geometry of the posterior surface 238b of the drive head 236, the drive head could be configured to simultaneously drive more or less than four arcuate fixation members 12C, to stagger the driving of one or more arcuate fixation members 12C, to distract one or more arcuate fixation members 12C, or any combination thereof.

In an example method of using the delivery instrument 208 to insert and secure an example assembly of the intervertebral implant system 100, an intervertebral implant body 108 may be nested an engaged within a fixation plate 116 to create an implant assembly. The intervertebral implant body 108 may be packed, for example with bone growth inducing substances, as appropriate. The distal ends 224b of the lateral arms 224 may be positioned to grip the fixation plate 116 and the lateral arms 224 may be locked in the gripping position. A plurality of arcuate fixation members 12C may be loaded into the guide ring 232 and the drive head 236 moved into position to drive the arcuate fixation members 12C. The delivery instrument 208 may then be used to position and insert the implant assembly into an intervertebral space. When the implant assembly has been positioned, the arcuate fixation members may be driven into position. The implant insertion process may be completed with the installation of a blocking plate 132 and a final tightening of a locking screw 138.

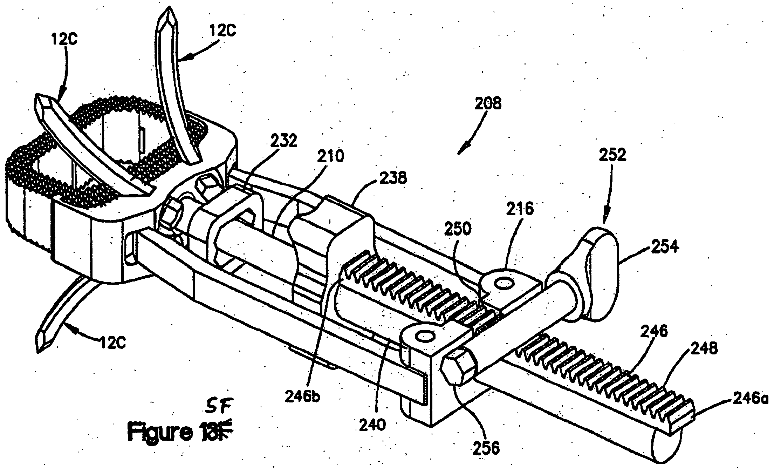

Various techniques may be utilized to actuate the drive head 236, thereby causing it to drive one or more arcuate fixation members 12C into position. In an example embodiment, the drive head 236 may be advanced towards the distal end 210b of drive shaft 210 by a series of impaction forces applied to the drive head 236. The impaction forces may be applied manually, for example by a surgeon via the use of a mallet, or mechanically, for example by a successive series of impactional strikes from a sonic hammer. In an alternative embodiment, the drive head 236 may be advanced via a mechanical drive system. For example, a rack and pinion system may be employed, as illustrated in Figure 5F. A rack 246 includes a proximal end 246a and a distal end 246b opposite the proximal end, the distal end 246b coupled to the drive head body 238 and/or the shaft 240. The rack may have a series of teeth 248 defined thereon between the proximal and distal ends 246a and 246b. The pivot block 216 may have a guide channel 250 formed within the upper side 218b of the body 218, the guide channel 250 configured to slidably receive the rack 246 therein. A pinion drive 252 may be coupled to the pivot block 216, for example on the anterior side 218a of the body 218, such that one or More pinion gears (not shown) of the pinion drive 252 engage the teeth 248 of the rack 246. The pinion drive 252 may be configured with a manual actuator 254 and/or an adapter 256, the adapter 256 configured to couple with a motorized source of rotational force. When the pinion drive 252 is actuated, via either the manual actuator 254 and/or a rotational force applied via the adapter 256, the rack 246, and consequently the drive head 236, may be translated in a direction toward the distal end 210b of the drive shaft 210, such that the arcuate fixation members 12C are driven into position.

Referring now to Figures 6A-6E, delivery instrument 278 includes a pair of insertion rods 258, each defining longitudinally opposed proximal and distal ends 258a and 258b. The distal ends 258b of the insertion rods 258 may be have engagement tips 258c formed thereon, the engagement tips 258c configured to be received by a fixation plate. In an example embodiment, the engagement tips 258c may define an outer diameter differing from the outer diameter of the distal ends 258b of the insertion rods 258, such that the outer diameter of the engagement tips 258c are uniquely sized to be received within the bores 178 of the fixation plate 156. The engagement tips 258c may have an engagement feature 258d formed thereon, the engagement feature 258d configured to releasably lock the distal ends 258b of the insertion rods 258 within the fixation plate 156. In an example embodiment, the engagement feature 258d may be a raised rib designed to engage with the retaining shelf 166 of the fixation plate 156 when a respective insertion rod 258 is inserted into the fixation plate 156 and rotated into a locked position. In alternative embodiments, the insertion rods 258 may be locked to the fixation plate utilizing other engagement features 258d, for example a ball and detent mechanism installed within the engagement tips 258c of the engagement rods 258, a series of gripping teeth defined on the engagement tip 258c, etc.

The insertion rods 258 may each have a channel 260 formed within the surface thereof between the proximal and distal ends 258a and 258b, the channels configured to directly oppose each other, facing inwardly, when the insertion rods 258 are locked in place within the fixation plate 156 in an assembled insertion configuration. The channels 260 may be configured to slidably receive lateral wings 276 coupled to the body 238 and/or the shaft 240 of the drive head 236, such that the drive head may translate between the proximal and distal ends 258a and 258b of the insertion rods 258 when the lateral wings are received with the channels 260. The channels 260 may be augmented by drop-in bores 262 formed within the outer surfaces of the insertion rods 258, the drop-in bores 262 configured to allow additional components of the intervertebral implant system 100, for example the drive 236 configured with the lateral wings 276, to be inserted into the channels 260 when the insertion rods 258 are already in an assembled configuration. Gripping features 264 may be formed within the outer surfaces of the insertion rods 258 between the proximal and distal ends 258a and 258b, the gripping features 264 configured to facilitate easier gripping of the insertion rods 258, for example as they are rotated into a locked position within the fixation plate 156. In an example embodiment, the gripping features 264 include a series of longitudinal grooves formed radially around the outer surfaces of the insertion rods, although any other gripping features may be used as appropriate. The proximal ends 258a of the insertion rods 258 may be configured to be received and lockably engaged within posterior bores 268 of an end block 266. For example the proximal ends 258a of the insertion rods may have one or more apertures (not shown) formed therethrough configured to accept a pin or other connector, may be have threads formed thereon configured to engage with complimentary threads formed within the end block 266, and so on.

The end block 266 may be defined by an anterior surface 266a, a posterior surface 266b opposite the anterior surface, lateral surfaces 266c, and upper and lower surfaces 266d and 266e. Posterior bores 268 may be formed within the posterior surface 266b of the block 266, the posterior bores 268 configured to receive the proximal ends 258a of the insertion rods 258. Opposing rod grooves 270 may be formed within the end block 266, the rod grooves 270 bounded by the lower and laterally facing quadrants of the posterior bores, extending outwardly to the lateral and bottom surfaces 226c and 266e respectively. The rod grooves 270 may be configured to allow the insertion of retaining members 272 through the rod grooves 270 and into the proximal ends 258a of the insertion rods 258. The retaining members 272 function to secure the insertion rods 258 within the posterior bores 268, while still permitting the rotation of the insertion rods 258, as limited by the lateral and lower facing surfaces of the rod grooves 270. The retaining members 272 may be, for example, pins, posts, screw-type anchors, or the like. An accessory bore 274 may be formed within the anterior surface 266a of the block 266, the accessory bore 274 configured to receive an accessory. The inner surface 274a of the accessory bore 274 may be configured with engaging features, for example helical threads, configured to engage complimentary engagement features formed on an accessory. Accessories may be configured to facilitate easier use of the delivery instrument 278 in positioning and/or insertion of an intervertebral implant assembly into an intervertebral space, and may include a "pistol" grip, a handle, a slap hammer, a slide hammer, or the like.

In an example method of using the delivery instrument 278 to insert and secure an example assembly of the intervertebral implant system 100, an intervertebral implant body 148 may mated with a fixation plate 156 to create an implant assembly. The intervertebral implant body 148 may be packed, for example with bone growth inducing substances, as appropriate. The engagement tips 258c of the insertion rods 258 may be inserted into and locked in place within the fixation plate 156. In an example embodiment, the insertion rods 258 are locked into place by first inserting the engagement tips 258c into the fixation plate, then rotating the insertion rods 258 one quarter turn to engage the raised ribs 258d with the retaining shelves 166 in the fixation plate 156. The delivery instrument 278 may then be used to position and insert the implant assembly into an intervertebral space. When the implant assembly has been positioned within the intervertebral space, the lateral wings 176 of the assembly carrying the retaining ring 232 and drive head 236, along with one or more pre-loaded arcuate fixation members 12C, may be placed into position in the channels 260 via the drop-in bores 262, and slid into position so that the arcuate fixation members 12C are aligned with the arcuate grooves of the fixation plate 156. The arcuate fixation members may then be driven into position. The implant insertion process may be completed with the installation of an optional blocking plate 180 and a final tightening of a locking screw 138.

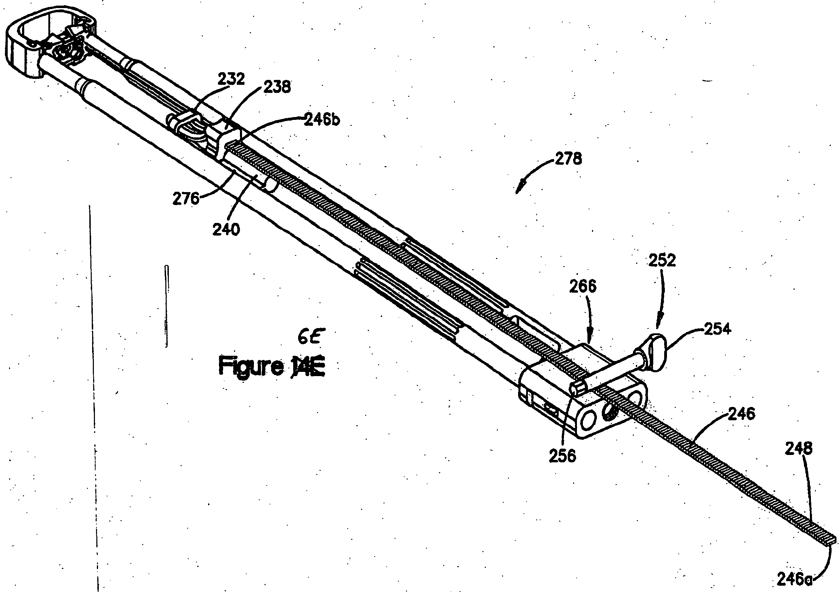

Various techniques may be utilized to actuate the drive head 236, thereby causing it to drive one or more arcuate fixation members 12C into position. In an example embodiment, the drive head 236 may be advanced towards the distal ends 258b of the insertion rods 258 by a series of impaction forces applied to the drive head 236. The impaction forces may be applied manually, for example by a surgeon via the use of a mallet, or mechanically, for example by a successive series of impactional strikes from a sonic hammer. In an alternative embodiment, the drive head 236 may be advanced via a mechanical drive system. For example, a rack and pinion system may be employed, as illustrated in Figure 6E. A rack 246 includes a proximal end 246a and a distal end 246b opposite the proximal end, the distal end 246b coupled to the drive head body 238 and/or the shaft 240. The rack may have a series of teeth 248 defined thereon between the proximal and distal ends 246a and 246b. The end block 266 may have a guise channel formed within the upper surface 266d, the guide channel configured to slidably receive the rack 246 therein. A pinion drive 252 may be coupled to the end block 266, for example on the upper surface 266d, such that one or more pinion gears (not shown) of the pinion drive 252 engage the teeth 248 of the rack 246. The pinion drive 252 may be configured with a manual actuator 254 and/or an adapter 256, the adapter 256 configured to couple with a motorized source of rotational force. When the pinion drive 252 is actuated, via either the manual actuator 254 and/or a rotational force applied via the adapter 256, the rack 246, and consequently the drive head 236, may be translated in a direction toward the distal ends 258b of the insertion rods 258, such that the arcuate fixation members 12C are driven into position.

It should be appreciated that a variety of kits can be provided that one or more components of the intervertebral implant system 100. The components of the kits may be configured the same or differently. For example, within a single kit, arcuate fixation members 12C may be provided that have varying lengths, differing radii of curvature, differing head and/or tab configurations, differing cross sectional geometries, and so on, depending for example on the type of procedure being performed by a surgeon, or on the particular anatomies of individual patients. The kits may also be configured differently with respect to which components of the individual systems are included in the kits. For example, a kit for the intervertebral implant system 100 may include arcuate fixation members 12C of varying lengths, radii of curvature, and/or features, and may include one or more of intervertebral implant bodies 108 or 148, fixation plates 116 or 156, blocking plates 132 or 180, ratchet plate 166, or locking screws 138. Example kits for the intervertebral implant system 100 may also include the respective delivery instruments 208, and/or 278.

Although arcuate fixation members and the other components of the intervertebral implant system 100 have been described herein with reference to preferred embodiments or preferred methods, it should be understood that the words which have been used herein are words of description and illustration, rather than words of limitation. For example, it should be noted that although the intervertebral implant system 100 has been described herein with reference to particular structure, methods, and/or embodiments, the scope of the instant disclosure is not intended to be limited to those particulars, but rather is meant to extend to all structures of the intervertebral implant system 100 which fall within the scope of the claims. Those skilled in the relevant art, having the benefit of the teachings of this specification, may effect numerous modifications to the intervertebral implant system 100 as described herein, and changes may be made without departing from the scope of the instant disclosure, for instance as recited in the appended claims.

## Claims

1. An intervertebral implant comprising:
an implant body (108; 148) defining a first bone-engaging surface (108d; 148d) and an opposed second bone-engaging surface (108e; 148e);
a fixation plate (116; 156) attached to the implant body (108; 148), the fixation plate (116; 156) defining at least one aperture (128; 176) extending therethrough; and
at least one curved fixation member (12C) configured to be received within the at least one aperture (128; 176), the at least one curved fixation member (12C) having a proximal end (102a) and a distal end (102b) opposite the proximal end (102a), the distal end (102b) comprising a tip (104) configured to cut into a vertebral body, the at least one curved fixation member (12C) having a length greater than the at least one aperture (128; 176) such that the tip (104) can be driven through the at least one aperture (128; 176) and into the vertebral body,
**characterized in that** the at least one aperture (128; 176) is curved.

2. The intervertebral implant as recited in claim 1, wherein the fixation plate (116) comprises an implant cradle defined by an upper surface (118b), a lower surface (118c), and an anterior surface (118a), the implant cradle configured to receive at least a portion of the implant body (108) in nested attachment.

3. The intervertebral implant as recited in claim 2, wherein the fixation plate further comprises first and second lateral arms extending away from the anterior surface (118a), the first and second lateral arms configured to releasably grasp the implant body (108).

4. The intervertebral implant as recited in claim 2 or 3, wherein the anterior surface (118a) has a concave recess (130) formed therein, the at least one curved aperture (128) defined within the concave recess (130), the concave recess (130) configured to matably engage with a blocking plate (132).

5. The intervertebral implant as recited in any one of claims 1 to 4, wherein the fixation plate (116) has an aperture (122) formed therethrough, the aperture (122) configured to releasably engage with a delivery instrument.

6. The intervertebral implant as recited in any one of claims 1 to 5, wherein at least one of the first bone-engaging surface (108d) or the second bone-engaging surface (108e) have a plurality of gripping structures (108h) formed thereon, the gripping structures (108h) configured to engage adjacent vertebral bodies when the implant body (108) is inserted into an intervertebral space.

7. The intervertebral implant as recited in any one of claims 1 to 6, wherein the implant body (108) defines at least one curved groove (112) formed thereon, the at least one curved groove (112) configured to receive the at least one curved fixation member (12C) when the intervertebral implant (100) is in an assembled configuration.

8. The intervertebral implant as recited in any one of claims 1 to 7, wherein the proximal end (102a) of the at least one curved fixation member (12C) comprises a locking feature (198; 200) that is configured to be lockably engaged within a complimentary locking feature (194) formed within the at least one curved aperture (128; 176) when the intervertebral implant is in an assembled configuration, wherein the locking feature (198) preferably is formed by teeth (198) disposed between the proximal end (102a) and the distal end (102b) of the at least one curved fixation member (12C).

9. The intervertebral implant as recited in any one of claims 1 to 8, wherein the fixation plate (116; 156) further defines a plurality of curved apertures (128; 176) extending therethrough disposed around a central bore (126; 174) defined through the fixation plate (116; 156), wherein preferably the plurality of curved apertures (128; 176) are disposed in opposing quadrants around the central bore (126; 174).

10. The intervertebral implant as recited in any one of claims 1 to 9, wherein the implant body (148) has a posterior side (148a) and lateral sides (148b) that terminate in distal ends (148c) opposite the posterior side (148a), and a height of the implant body (148), measured between the first and second bone-engaging surfaces (148d, 148e), increases from the posterior side (148a) in a direction toward the distal ends (148c).

11. An intervertebral implant system, comprising:
an intervertebral implant as recited in any one of claims 1 to 10, and
a delivery instrument (208; 278) comprising:
a pair of insertion rods (224; 258) comprising proximal ends (224a; 258a) and distal ends (224b; 258b) opposite the proximal ends (224a; 258a), the distal ends (224b; 258b) configured to releasably grip the fixation plate (116; 156);
a guide ring (232) having an inner surface (234c) with a plurality of curved guiding slots (234d) formed therein, the curved guiding slots (234d) configured to guide the plurality of curved fixation members (12C) into the curved apertures (128; 176); and
a drive head (236) configured to impart a force to at least one of the plurality of curved fixation members (12C) that biases the tip (104) of the at least one of the plurality of curved fixation members (12C) into the vertebral body, wherein the drive head (236) preferably has a plurality of curved receptors formed therein, each of the curved receptors configured to releasably receive the proximal end (102a) of a respective one of the plurality of curved fixation members (12C).

12. The intervertebral implant system as recited in claim 11, wherein the delivery instrument (208) further comprises:
a drive shaft (210) having a first proximal end (210a) and a first distal end (210b) opposite the first proximal end (210a), the drive shaft (210) configured to carry the guide ring (232) and the drive head (236), the first distal end (210b) comprising a threaded portion (212) configured to releasably engage a threaded bore (126) in the fixation plate (116); and
a pivot block (216) affixed to the drive shaft (210), the pivot block (216) having a pair of pivot points defined thereon configured to receive the pair of insertion rods (224) in pivoting engagement.

13. The intervertebral implant system as recited in claim 12, wherein:
the pivot block (216) is configured to slidably receive a driving rack (246), the pivot block (216) further having a pinion drive (252) affixed thereto, the pinion drive (252) configured to drivingly engage the driving rack (246); and
wherein the driving rack (246) is affixed to the drive head (236), such that when the pinion drive (252) applies a driving force to the driving rack (246), the driving rack (246) advances the drive head (236) along the drive shaft (210) in a direction towards the fixation plate (116), thereby imparting the force to the at least one of the plurality of curved fixation members (12C).

14. The intervertebral implant system as recited in claim 11, wherein:
the pair of insertion rods (258) further comprise a pair of longitudinal slots (260) formed therein between the proximal ends (258a) and distal ends (258b); and
wherein the delivery instrument (278) further comprises:
a mounting block (266) affixed to the proximal ends (258a) of the pair of insertion rods (258); and
a carriage having a pair of lateral wings (276) configured to be slidably received within the longitudinal slots (260), the carriage configured to carry the guide ring (232) and the drive head (236).

15. The intervertebral implant system as recited in claim 14, wherein:
the mounting block (266) is configured to slidably receive a driving rack (246), the mounting block (266) further having a pinion drive (252) affixed thereto, the pinion drive (252) configured to drivingly engage the driving rack (246); and
wherein the driving rack (246) is affixed to the carriage, such that when the pinion drive (252) applies a driving force to the driving rack (246), the driving rack (246) advances the carriage in a longitudinal direction towards the fixation plate (156), thereby imparting the force to the at least one of the plurality of curved fixation members (12C),

## Patentansprüche

1. Zwischenwirbelimplantat, umfassend:
einen Implantatkörper (108; 148), der eine erste Knocheneingriffsoberfläche (108d; 148d) und eine gegenüberliegende zweite Knocheneingriffsoberfläche (108e; 148e) definiert,
eine an dem Implantatkörper (108; 148) angebrachte Fixierplatte (116; 156), wobei die Fixierplatte (116; 156) zumindest eine sich dort hindurch erstreckende Öffnung (128; 176) definiert, und
zumindest ein gekrümmtes Fixierelement (12C), das eingerichtet ist, in der zumindest einen Öffnung (128; 176) aufgenommen zu werden, wobei das zumindest eine gekrümmte Fixierelement (12C) ein proximales Ende (102a) und ein distales Ende (102b) gegenüber dem proximalen Ende (102a) aufweist, wobei das distale Ende (102b) eine Spitze (104) umfasst, die eingerichtet ist, in einen Wirbelkörper zu schneiden, wobei das zumindest eine gekrümmte Fixierelement (12C) eine Länge aufweist, die größer ist als die zumindest eine Öffnung (128; 176), so dass die Spitze (104) durch die zumindest eine Öffnung (128; 176) und in den Wirbelkörper hinein getrieben werden kann,
**dadurch gekennzeichnet, dass** die zumindest eine Öffnung (128; 176) gekrümmt ist.

2. Zwischenwirbelimplantat nach Anspruch 1, wobei die Fixierplatte (116) ein Implantatlager umfasst, das durch eine obere Oberfläche (118b), eine untere Oberfläche (118c) und eine anteriore Oberfläche (118a) definiert ist, wobei das Implantatlager eingerichtet ist, zumindest einen Teil des Implantatkörpers (108) in einer ineinander gesteckten Anbringung aufzunehmen.

3. Zwischenwirbelimplantat nach Anspruch 2, wobei die Fixierplatte des Weiteren einen ersten und zweiten lateralen Arm umfasst, die sich weg von der anterioren Oberfläche (118a) erstrecken, wobei der erste und zweite laterale Arm eingerichtet sind, den Implantatkörper (108) lösbar zu greifen.

4. Zwischenwirbelimplantat nach Anspruch 2 oder 3, wobei die anteriore Oberfläche (118a) eine darin gebildete konkave Ausnehmung (130) aufweist, wobei die zumindest eine gekrümmte Öffnung (128) in der konkaven Ausnehmung (130) definiert ist, wobei die konkave Ausnehmung (130) eingerichtet ist, mit einer Blockierplatte (132) passend zusammenzuwirken.

5. Zwischenwirbelimplantat nach einem der Ansprüche 1 bis 4, wobei die Fixierplatte (116) eine dort hindurch gebildete Öffnung (122) aufweist, wobei die Öffnung (122) eingerichtet ist, lösbar mit einem Zuführinstrument zusammenzuwirken.

6. Zwischenwirbelimplantat nach einem der Ansprüche 1 bis 5, wobei zumindest eine der ersten Knocheneingriffsoberfläche (108d) oder der zweiten Knocheneingriffsoberfläche (108e) eine Mehrzahl von darauf gebildeten Greifstrukturen (108h) aufweist, wobei die Greifstrukturen (108h) eingerichtet sind, mit benachbarten Wirbelkörpern zusammenzuwirken, wenn der Implantatkörper (108) in einen Zwischenwirbelraum eingeführt ist.

7. Zwischenwirbelimplantat nach einem der Ansprüche 1 bis 6, wobei der Implantatkörper (108) zumindest eine darauf gebildete gekrümmte Nut (112) definiert, wobei die zumindest eine gekrümmte Nut (112) eingerichtet ist, das zumindest eine gekrümmte Fixierelement (12C) aufzunehmen, wenn das Zwischenwirbelimplantat (100) in einem zusammengebauten Zustand ist.

8. Zwischenwirbelimplantat nach einem der Ansprüche 1 bis 7, wobei das proximale Ende (102a) des zumindest einen gekrümmten Fixierelements (12C) ein Verriegelungsmerkmal (198; 200) umfasst, das eingerichtet ist, verriegelbar mit einem in der zumindest einen gekrümmten Öffnung (128; 176) gebildeten komplementären Verriegelungsmerkmal (194) zusammenzuwirken, wenn das Zwischenwirbelimplantat in einem zusammengebauten Zustand ist, wobei das Verriegelungsmerkmal (198) vorzugsweise durch Zähne (198) gebildet ist, die zwischen dem proximalen Ende (102a) und dem distalen Ende (102b) des zumindest einen gekrümmten Fixierelements (12C) angeordnet sind.

9. Zwischenwirbelimplantat nach einem der Ansprüche 1 bis 8, wobei die Fixierplatte (116; 156) des Weiteren eine Mehrzahl von sich dort hindurch erstreckenden gekrümmten Öffnungen (128; 176) definiert, die um eine durch die Fixierplatte (116; 156) definierte zentrale Bohrung (126; 174) herum angeordnet sind, wobei die Mehrzahl von gekrümmten Öffnungen (128; 176) vorzugsweise in gegenüberliegenden Quadranten um die zentrale Bohrung (126; 174) herum angeordnet sind.

10. Zwischenwirbelimplantat nach einem der Ansprüche 1 bis 9, wobei der Implantatkörper (148) eine posteriore Seite (148a) und laterale Seiten (148b) aufweist, die an distalen Enden (148c) gegenüber der posterioren Seite (148a) enden, und eine Höhe des Implantatkörpers (148), gemessen zwischen der ersten und zweiten Knocheneingriffsoberfläche (148d, 148e), von der posterioren Seite (148a) in einer Richtung zu den distalen Enden (148c) hin ansteigt.

11. Zwischenwirbelimplantatsystem, umfassend:
ein Zwischenwirbelimplantat nach einem der Ansprüche 1 bis 10 und
ein Zuführinstrument (208; 278), umfassend:
ein Paar Einführstangen (224; 258), die proximale Enden (224a; 258a) und distale Enden (224b; 258b) gegenüber den proximalen Enden (224a; 258a) umfassen, wobei die distalen Enden (224b; 258b) eingerichtet sind, die Fixierplatte (116; 156) lösbar zu greifen,
einen Führungsring (232), der eine innere Oberfläche (234c) mit einer Mehrzahl von darin gebildeten gekrümmten Führungsschlitzen (234d) aufweist, wobei die Führungsschlitze (234d) eingerichtet sind, die Mehrzahl von gekrümmten Fixierelementen (12C) in die gekrümmten Öffnungen (128; 176) hinein zu führen, und
einen Treibkopf (236), der eingerichtet ist, eine Kraft auf zumindest eines der Mehrzahl von Fixierelementen (12C) zu übermitteln, die die Spitze (104) des zumindest einen der Mehrzahl von gekrümmten Fixierelementen (12C) in den Wirbelkörper hinein drängt, wobei der Treibkopf (236) vorzugsweise eine Mehrzahl von darin gebildeten gekrümmten Aufnahmen aufweist, wobei jede der gekrümmten Aufnahmen eingerichtet ist, das proximale Ende (102a) eines entsprechenden der Mehrzahl von gekrümmten Fixierelementen (12C) lösbar aufzunehmen.

12. Zwischenwirbelimplantatsystem nach Anspruch 11, wobei das Zuführinstrument (208) des Weiteren umfasst:
einen Treibschaft (210), der ein erstes proximales Ende (210a) und ein erstes distales Ende (210b) gegenüber dem ersten proximalen Ende (210a) aufweist, wobei der Treibschaft (210) eingerichtet ist, den Führungsring (232) und den Treibkopf (236) zu tragen, wobei das erste distale Ende (210b) einen Teil (212) mit Gewinde umfasst, der eingerichtet ist, mit einer Bohrung (126) mit Gewinde in der Fixierplatte (116) lösbar zusammenzuwirken, und
einen am Treibschaft (210) befestigten Schwenkblock (216), wobei der Schwenkblock (216) ein Paar darauf definierte Schwenkpunkte aufweist, die eingerichtet sind, das Paar Einführstangen (224) in schwenkendem Eingriff aufzunehmen.

13. Zwischenwirbelimplantatsystem nach Anspruch 12, wobei:
der Schwenkblock (216) eingerichtet ist, eine Antriebsschiene (246) verschieblich aufzunehmen, wobei der Schwenkblock (216) des Weiteren einen daran befestigten Zahnradantrieb (252) aufweist, wobei der Zahnradantrieb (252) eingerichtet ist, mit der Antriebsschiene (246) antreibend zusammenzuwirken, und
wobei die Antriebsschiene (246) an dem Treibkopf (236) befestigt ist, so dass, wenn der Zahnradantrieb (252) eine Antriebskraft auf die Antriebsschiene (246) ausübt, die Antriebsschiene (246) den Treibkopf (236) entlang des Treibschafts (210) in einer Richtung zur Fixierplatte (116) hin voran bewegt, wodurch die Kraft auf das zumindest eine der Mehrzahl von gekrümmten Fixierelementen (12C) übermittelt wird.

14. Zwischenwirbelimplantatsystem nach Anspruch 11, wobei:
das Paar Einführstangen (258) des Weiteren ein Paar längliche Schlitze (260) umfasst, die darin zwischen den proximalen Enden (258a) und distalen Enden (258b) gebildet sind, und
wobei das Zuführinstrument (278) des Weiteren umfasst:
einen an den proximalen Enden (258a) des Paars Einführstangen (258) befestigten Halterungsblock (266), und
einen Träger, der ein Paar laterale Flügel (276) aufweist, die eingerichtet sind, in den länglichen Schlitzen (260) verschieblich aufgenommen zu werden, wobei der Träger eingerichtet ist, den Führungsring (232) und den Treibkopf (236) zu tragen.

15. Zwischenwirbelimplantatsystem nach Anspruch 14, wobei:
der Halterungsblock (266) eingerichtet ist, eine Antriebsschiene (246) verschieblich aufzunehmen, wobei der Halterungsblock (266) des Weiteren einen daran befestigten Zahnradantrieb (252) aufweist, wobei der Zahnradantrieb (252) eingerichtet ist, mit der Antriebsschiene (246) antreibend zusammenzuwirken, und
wobei die Antriebsschiene (246) an dem Träger befestigt ist, so dass, wenn der Zahnradantrieb (252) eine Antriebskraft auf die Antriebsschiene (246) ausübt, die Antriebsschiene (246) den Träger in einer Längsrichtung zur Fixierplatte (156) hin voran bewegt, wodurch die Kraft auf das zumindest eine der Mehrzahl von gekrümmten Fixierelementen (12C) übermittelt wird.

## Revendications

1. Implant intervertébral, comprenant :
un corps d'implant (108 ; 148) définissant une première surface de prise avec l'os (108d; 148d) et une seconde surface de prise avec l'os opposée (108e; 148e) ;
une plaque de fixation (116 ; 156) fixée au corps d'implant (108 ; 148), la plaque de fixation (116; 156) définissant au moins une ouverture (128; 176) s'étendant à travers elle, et
au moins un élément de fixation courbe (12C) configuré pour être reçu dans la au moins une ouverture (128 ; 176), le au moins un élément de fixation courbe (12C) présentant une extrémité proximale (102a) et une extrémité distale (102b) face à l'extrémité proximale (102a), l'extrémité distale (102b) comprenant une pointe (104) configurée pour couper dans un corps vertébral, le au moins un élément de fixation courbe (12C) présentant une longueur supérieure à la au moins une ouverture (128; 176), de sorte que la pointe (104) peut être entraînée à travers la au moins une ouverture (128; 176) et dans le corps vertébral,
**caractérisé en ce que** la au moins une ouverture (128; 176) est courbe.

2. Implant intervertébral selon la revendication 1, dans lequel la plaque de fixation (116) comprend un arceau d'implant défini par une surface supérieure (118b), une surface inférieure (118c), et une surface antérieure (118a), l'arceau d'implant étant configuré pour recevoir au moins une portion du corps d'implant (108) en fixation emboîtée.

3. Implant intervertébral selon la revendication 2, dans lequel la plaque de fixation comprend en outre des premier et second bras latéraux s'étendant d'une manière s'éloignant de la surface antérieure (118a), les premier et second bras étant configurés pour saisir d'une manière pouvant être désolidarisée le corps d'implant (108).

4. Implant intervertébral selon la revendication 2 ou 3, dans lequel la surface antérieure (118a) présente un retrait concave (130) formé dans celle-ci, la au moins une ouverture courbe (128) étant définie dans le retrait concave (130), et le retrait concave (130) étant configuré pour venir en prise par appariement avec une plaque de blocage (132).

5. Implant intervertébral selon l'une quelconque des revendications 1 à 4, dans lequel la plaque de fixation (116) présente une ouverture (122) à travers elle, l'ouverture (122) étant configurée pour venir en prise d'une manière pouvant être désolidarisée avec un instrument de fourniture.

6. Implant intervertébral selon l'une quelconque des revendications 1 à 5, dans lequel au moins une parmi la première surface de prise avec l'os (108d) ou une seconde surface de prise avec l'os (108e) présente une pluralité de structures de prise (108h) formées sur celles-ci, les structures de prise (108h) étant configurées pour venir en prise avec des corps vertébraux adjacents lorsque le corps d'implant (108) est introduit dans un espace intervertébral.

7. Implant intervertébral selon l'une quelconque des revendications 1 à 6, dans lequel le corps d'implant (108) définit au moins une rainure courbe (112) formée sur lui, la au moins une rainure courbe (112) étant configurée pour recevoir la au moins une élément de fixation courbe (12C) lorsque l'implant intervertébral (100) est dans une configuration assemblée.

8. Implant intervertébral selon l'une quelconque des revendications 1 à 7, dans lequel l'extrémité proximale (102a) du au moins un élément de fixation courbe (12C) comprend une fonctionnalité de blocage (198; 200) configurée pour venir en prise d'une manière permettant le blocage dans une fonctionnalité de blocage complémentaire (194), formée dans la au moins une ouverture courbe (128 ; 176), lorsque l'implant intervertébral se trouve dans une configuration assemblée, dans lequel la fonctionnalité de blocage (198) est de préférence formée par des dents (198) disposées entre l'extrémité proximale (102a) et l'extrémité distale (102b) du au moins un élément de fixation courbe (12C).

9. Implant intervertébral selon l'une quelconque des revendications 1 à 8, dans lequel la plaque de fixation (116; 156) définit en outre une pluralité d'ouvertures courbes (128; 176) s'étendant à travers elle, disposées autour d'un alésage central (126; 174) défini à travers la plaque de fixation (116 ; 156), dans lequel la pluralité d'ouvertures courbes (128; 176) sont de préférence disposées dans des quadrants opposés autour de l'alésage central (126; 174).

10. Implant intervertébral selon l'une quelconque des revendications 1 à 9, dans lequel le corps d'implant (148) présente un côté postérieur (148a) et des côtés latéraux (148b) qui se terminent en des extrémités distales (148c) face au côté postérieur (148a), et une hauteur du corps d'implant (148), mesurée entre les première et seconde surfaces de prise avec l'os (148d, 148e), augmente à partir du côté postérieur (148a) dans une direction allant vers les extrémités distales (148c).

11. Système d'implant intervertébral comprenant :
un implant intervertébral selon l'une quelconque des revendications 1 à 10, et
un instrument de fourniture (208; 278) comprenant :
une paire de tiges d'introduction (224; 258) comprenant des extrémités proximales (224a; 258a) et des extrémités distales (224b; 258b) face aux extrémités proximales (224a; 258a), les extrémités distales (224b; 258b) étant configurées pour prendre d'une manière pouvant être désolidarisée la plaque de fixation (116; 156) ;
un anneau de guidage (232) présentant une surface intérieure (234c) dotée d'une pluralité de fentes de guidage courbes (234d) formées dans celle-ci, les fentes de guidage courbes (234d) étant configurées pour guider la pluralité d'éléments de fixation courbes (12C) dans les ouvertures courbes (128; 176), et
une tête d'entraînement (236) configurée pour impartir une force au au moins un élément parmi la pluralité d'éléments de fixation courbes (12C) qui sollicite la pointe (104) du au moins un élément parmi la pluralité d'éléments de fixation courbes (12C) jusque dans le corps vertébral, dans lequel la tête d'entraînement (236) présente de préférence une pluralité de récepteurs courbes formés dedans, chacun des récepteurs courbes étant configuré pour recevoir d'une manière pouvant être désolidarisée l'extrémité proximale (102a) d'un élément respectif parmi la pluralité d'éléments de fixation courbes (12C).

12. Système d'implant intervertébral selon la revendication 11, dans lequel l'instrument de fourniture (208) comprend en outre :
un arbre d'entraînement (210) présentant une première extrémité proximale (210a) et une première extrémité distale (210b) face à la première extrémité proximale (210a), l'arbre d'entraînement (210) étant configuré pour porter l'anneau de guidage (232) et la tête d'entraînement (236), la première extrémité distale (210b) comprenant une portion filetée (212) configurée pour venir en prise d'une manière pouvant être désolidarisée avec un alésage fileté (126) dans la plaque de fixation (116), et
un bloc de pivotement (216) fixé sur l'arbre d'entraînement (210), le bloc de pivotement (216) présentant une paire de points de pivotement définis sur lui configurés pour recevoir la paire de tiges d'introduction (224) dans une prise par pivotement.

13. Système d'implant intervertébral selon la revendication 12, dans lequel :
le bloc de pivotement (216) est configuré pour recevoir par coulissement une crémaillère d'entraînement (246), le bloc de pivotement (216) présentant en outre un entraînement à pignon (252) fixé sur lui, l'entraînement à pignon (252) étant configuré pour venir en prise par entraînement avec la crémaillère d'entraînement (246), et
dans lequel la crémaillère d'entraînement (246) est fixée à la tête d'entraînement (236), de telle sorte que lorsque l'entraînement à pignon (252) applique une force d'entraînement à la crémaillère d'entraînement (246), la crémaillère d'entraînement (246) fait avancer la tête d'entraînement (236) le long de l'arbre d'entraînement (210) dans un direction allant vers la plaque de fixation (116), impartissant ainsi la force au au moins un élément parmi la pluralité d'éléments de fixation courbes (12C).

14. Système d'implant intervertébral selon la revendication 11, dans lequel :
la paire de tiges d'introduction (258) comprend en outre une paire de fentes longitudinales (260) formées dedans entre les extrémités proximales (258a) et les extrémités distales (258b), et
dans lequel l'instrument de fourniture (278) comprend en outre :
un bloc de montage (266) fixé aux extrémités proximales (258a) de la paire de tiges d'introduction (258), et
un chariot présentant une paire d'ailes latérales (276) configurées pour être reçues par coulissement dans les fentes longitudinales (260), le chariot étant configuré pour porter l'anneau de guidage (232) et la tête d'entraînement (236).

15. Système d'implant intervertébral selon la revendication 14, dans lequel :
le bloc de montage (266) est configuré pour recevoir par coulissement une crémaillère d'entraînement (246), le bloc de montage (266) présentant en outre un entraînement à pignon (252) fixé sur lui, l'entraînement à pignon (252) étant configuré pour venir en prise par entraînement avec la crémaillère d'entraînement (246), et
dans lequel la crémaillère d'entraînement (246) est fixée sur le chariot, de sorte que lorsque l'entraînement à pignon (252) applique une force d'entraînement à la crémaillère d'entraînement (246), la crémaillère d'entraînement (246) fait avancer le chariot dans une direction longitudinale allant vers la plaque de fixation (156), impartissant ainsi la force au au moins un élément parmi la pluralité d'éléments de fixation courbes (12C).
